(19) Europäisches Patentamt
European Patent Office
Office européen des brevets

(11) **EP 1 450 832 B1**

(12) **FASCICULE DE BREVET EUROPEEN**

(45) Date de publication et mention
de la délivrance du brevet:
**16.08.2006 Bulletin 2006/33**

(51) Int Cl.:
***A61K 35/74*** *(2006.01)*

(21) Numéro de dépôt: **02781021.7**

(22) Date de dépôt: **27.11.2002**

(86) Numéro de dépôt international:
**PCT/CA2002/001826**

(87) Numéro de publication internationale:
**WO 2003/045405 (05.06.2003 Gazette 2003/23)**

(54) **BACTERIES LACTIQUES ET LEUR USAGE DANS LE TRAITEMENT ET LA PREVENTION DU CANCER**

MILCHSÄUREBAKTERIEN UND DEREN VERWENDUNGEN ZUR BEHANDLUNG VON UND VORBEUGUNG GEGEN KREBS

LACTIC ACID BACTERIA AND THEIR USE FOR TREATING AND PREVENTING CANCER

(84) Etats contractants désignés:
**AT BE BG CH CY CZ DE DK EE ES FI FR GB GR IE IT LI LU MC NL PT SE SK TR**

(30) Priorité: **27.11.2001 CA 2364249**

(43) Date de publication de la demande:
**01.09.2004 Bulletin 2004/36**

(73) Titulaire: **Bio-K Plus International, Inc.**
**Laval,**
**Québec H7V 4A7 (CA)**

(72) Inventeurs:
• **LUQUET, François-Marie**
**F-91400 Orsay Paris (FR)**
• **BALDWIN, Cindy**
**Laval, Québec H7G 4Y6 (CA)**
• **LACROIX. Monique**
**St-Lambert, Québec J4P 1T4 (CA)**

(74) Mandataire: **Goulard, Sophie et al**
**Cabinet ORES,**
**36, rue de St Pétersbourg**
**75008 Paris (FR)**

(56) Documents cités:
• **HIRAYAMA KAZUHIRO ET AL: "The role of probiotic bacteria in cancer prevention." MICROBES AND INFECTION, vol. 2, no. 6, mai 2000 (2000-05), pages 681-686, XP002239621 ISSN: 1286-4579**

• **WOLLOWSKI INGRID ET AL: "Protective role of probiotics and prebiotics in colon cancer." AMERICAN JOURNAL OF CLINICAL NUTRITION, vol. 73, no. 2S, février 2001 (2001-02), pages 451S-455S, XP002239622 International Symposium on Probiotics and Prebiotics;Kiel, Germany; June 11-12, 1998 ISSN: 0002-9165**

• **KAILASAPATHY KAILA ET AL: "Survival and therapeutic potential of probiotic organisms with reference to Lactobacillus acidophilus and Bifidobacterium spp." IMMUNOLOGY AND CELL BIOLOGY, vol. 78, no. 1, février 2000 (2000-02), pages 80-88, XP002239623 ISSN: 0818-9641**

• **HAGUE A ET AL: "The short-chain fatty acid butyrate induces apoptosis in colorectal tumour cell lines." EUROPEAN JOURNAL OF CANCER PREVENTION: THE OFFICIAL JOURNAL OF THE EUROPEAN CANCER PREVENTION ORGANISATION (ECP). ENGLAND OCT 1995, vol. 4, no. 5, octobre 1995 (1995-10), pages 359-364, XP009010019 ISSN: 0959-8278**

• **MARCHETTI M C ET AL: "POSSIBLE MECHANISMS INVOLVED IN APOPTOSIS OF COLON TUMOR CELL LINES INDUCED BY DEOXYCHOLIC ACID, SHORT-CHAIN FATTY ACIDS, AND THEIR MIXTURES" NUTRITION AND CANCER, LONDON, GB, vol. 28, no. 1, 1997, pages 74-80, XP009010020 ISSN: 0163-5581**

**Description**

**DOMAINE DE L'INVENTION**

La présente invention concerne la mise en évidence de l'utilité de la souche bactérienne lactique Lactobacillus acidophilus I-1492 déposée à la CNCM dans la prévention et le traitement d'un cancer. Plus particulièrement, la présente invention concerne l'usage de ladite bactérie lactique pour faciliter l'induction de l'apoptose cellulaire d'un cancer.

**DESCRIPTION DE L'ART ANTÉRIEUR**

Les bactéries Lactobacillus acidophilus I-1492 présentes dans le produit Bio-K + et qui font l'objet de la demande de brevet WO 98/23727 sont reconnus pour avoir un effet bénéfique sur le taux de cholestérol sanguin chez les mammifères.

La demande internationale no. WO 98/23727 a pour objet un ferment lactique qui comprend une souche de Lactobacillus acidophilus I-1492. Ainsi, ces bactéries sont principalement utilisées pour la préparation de ferment lactique pour réduire le taux de cholestérol sanguin chez les mammifères.

De plus, ces bactéries sont également reconnues pour avoir des propriétés qui ont pour effet de renforcer le système immunitaire, de faciliter l'absorption des nutriments et de stimuler la flore intestinale. Il est connu que les bactéries lactiques ont un effet positif sur la flore intestinale et également sur le système immunitaire. En effet, les bactéries lactiques permettent une stimulation du système immunitaire ayant donc pour effet de donner une meilleure défense au niveau du système digestif. Ces bactéries sont également connues pour neutraliser les effets secondaires causés par divers antibiotiques.

Au pays, un bon nombre de personnes meurent chaque année du cancer du colon. Le cancer est la troisième maladie qui cause le plus de mort par année. Au Canada, en l'an 2000, le taux de mortalité causé par le cancer était de 6500 et il y a plus de 17 000 nouveaux cas.

L'usage de nutraceutique impliquant l'administration de yogourt et/ou de lait fermenté comme traitement complémentaire contre le cancer est également connu.

Le traitement déjà utilisé comme thérapie chez l'homme, est l'ablation de la masse cancéreuse par chirurgie et ensuite il peut y avoir irradiation de la zone où se trouvait le cancer afin d'éviter de laisser des traces des cellules indésirables.

Il existe également la chimiothérapie. Ce traitement implique l'administration d'agents anticancéreux tels que le 5 fluoro-uracile (5FU). Ce composé est combiné avec un adjuvant pour limiter les effets négatifs de la chimiothérapie. Le 5 fluoro-uracile est un médicament qui est couramment utilisé pour traiter le cancer du colon. Ce médicament peut être administré de façon orale ou intra péritonéale (le plus près de la cible cancéreuse) vu sa grande instabilité dans le sérum. Il est connu pour causer la mort des cellules cancéreuses du colon et ce par différents moyens.

La mort d'une cellule, que l'on nomme aussi apoptose, peut s'exécuter par l'intermédiaire de différentes protéines. A titre d'exemple, l'apoptose cellulaire peut résulter de l'activation d'un récepteur membranaire ou de l'expression cytoplasmique de différentes protéines favorisant ce phénomène. A ce sujet, il est connu que le 5FU augmente l'expression de la protéine p53 ainsi que l'expression du récepteur membranaire Fas.

**1 Généralité sur l'apoptose**

**1.1 Définition de l'apoptose**

Découvert et redécouvert plusieurs fois par différents cytologistes et biologistes, la mort cellulaire programmée à acquis différent nom au court des deux derniers siècles. En 1972, le terme apoptose à finalement été adopté et inventé par Currie et c'est collègues, afin de décrire un modèle fréquent de mort cellulaire programmée que les auteurs ont observé de façon répété dans plusieurs tissus et types de cellules. Il a été observé que ces cellules mourantes partageaient plusieurs caractéristiques morphologiques, qui sont différentes des caractéristiques observées sur des cellules atteintes d'une pathologie et des cellules nécrosées, et ils suggèrent que ces caractéristiques morphologiques partagées pourraient être le résultat d'un programme de mort cellulaire commun et conservé.

**1.2 Rôle de l'apoptose**

Les chercheurs ont découvert que les cellules de notre corps peuvent se donner la mort, ils savent que ce « suicide » cellulaire, appelé « apoptose » est indispensable à l'organisme. L'apoptose est aussi fondamentale pour la physiologie des cellules et des tissus que la division et la différentiation cellulaire. L'apoptose est la forme de mort cellulaire physiologique la plus commune qui se produit dans différents moments comme par exemple, lors du développement embryonnaire, lors de la réorganisation tissulaire, lors des régulations immunitaires et de la régression tumorale. Ainsi, la mort cellulaire physiologique est un processus d'élimination cellulaire spontané, permettant d'assurer le renouvellement cellulaire, et qui intervient dans le maintien de l'homéostasie cellulaire et tissulaire de façon opposée à la mitose. Elle est le mécanisme inné par lequel l'organisme élimine les cellules indésirables. Chaque cellule porte en elle le mécanisme génétique de sa propre destruction. La cellules ne restent en vie qu'à condition de recevoir des signaux de survie émis par son environnement. Si la cellule perçoit des signaux lui ordonnant de se suicider, alors elle déclenche le programme de mort. Un dérèglement au niveau de l'équilibre entre les protéines maintenant la cellule en vie et entre les protéines menant à la mort des cellules peut être associé à un large spectre de maladie comprenant le cancer, la neurodégénération, des maladies auto-immunes, au diabète et d'autres désordres.

## 2 Caractéristiques morphologiques de l'apoptose et de la nécrose

### 2.1 Apoptose

Une des caractéristiques clé de l'apoptose est le rétrécissement cellulaire. Pendant que le rétrécissement cellulaire se produit, le cytoplasme se comprime et la chromatine nucléaire se condense et forme des agrégats, dans le noyaux, qui se collent ensuite contre la membrane nucléaire. Les organites cellulaires, dont la mitochondrie, en revanche, semblent relativement inchangées. Par la suite, le noyaux devient fragmenté. La formation et l'émission de bourgeons sont observées à la surface de la cellule. L'intégrité de la membrane plasmique, même si la perméabilité augmente, est cependant conservée tout au long de ce processus. Lors de l'étape finale de l'apoptose, la cellule se brisé en plusieurs vésicules contenant une variété d'organelles intacts et de fragments nucléaires. Les fragments de cellules apoptotiques sont rapidement engouffrées par les cellules phagocytaires environnantes, comme exemple le macrophage. L'apoptose constitue une mort dite « propre » puisque les fragments cellulaires sont rapidement éliminés. Il n'y a ni phase inflammatoire, ni lésion du tissu environnant et ce en partie parce que leur membrane cellulaire reste intact. En résumé, les changements morphologiques caractéristiques à l'apoptose sont le rétrécissement du cytoplasme, la condensation et la fragmentation de l'ADN et finalement, la formation de corps apoptotiques renferment les fragments de noyau entouré du cytoplasme et de la membrane cellulaire.

### 2.2 Nécrose

La nécrose fait référence à une mort soudaine intervenant suite à un stress physique ou chimique extrême. Elle est marquée par des critères morphologiques différents. Lors de la nécrose, cette mort cellulaire incontrôlée, il y a rapidement perte du contrôle du flux ionique entraînant la pénétration de l'eau et une augmentation de l'influx ionique, les cellules gonflent ainsi que ses organites comme la mitochondrie et le réticulum endoplasmique jusqu'à l'éclatement des membranes et la fragmentation non spécifique de l'ADN dans le noyau. Le relâchement du contenu cytoplasmique à l'extérieur à l'ajout de d'autres événements, provoque le plus souvent des lésions dans les tissus situés à proximité et déclenche une réponse inflammatoire locale très prononcée.

**Tableau 1- Les principales différences entre la nécrose et l'apoptose.**

| Caractéristiques | Nécrose | Apoptose |
|---|---|---|
| Distribution tissulaire | regroupement cellulaire | cellule isolée |
| Réaction tissulaire | La lyse et le relâchement du contenu cellulaire aboutissant à l'inflammation des tissus environnants | Phagocytose des corps apoptotiques par les macrophages ou les cellules environnantes et aucune inflammation |
| Morphologie Cellule | Gonflement | Rétrécissement, perte de contact avec les cellules environnantes, "blebbing", formation de corps apoptotique |
| Organelles | Endommagées | Intactes |
| Noyau | Désintégré | Condensé et fragmenté |
| Lysosomes | Endommagés | Intactes |
| Mitochondries | Défectueuses, épuisées en ATP, enflées et endommagées | Enflées, changement peuvent se rompre, relâchement de cytochrome C |
| Biochimie ADN | dégradation non spécifique | clivage à l'intérieur du noyau |

(suite)

| Caractéristiques | Nécrose | Apoptose |
|---|---|---|
| Protéines | dégradation non spécifique | Activation des caspases |

### 3 Différentes étapes du processus apoptotique

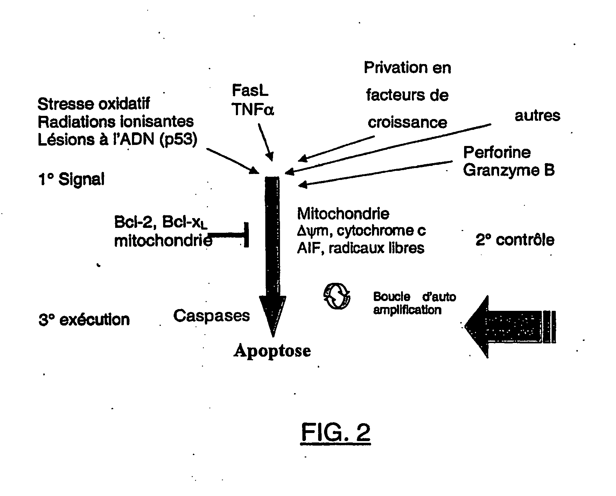

L'apoptose peut comporter trois différentes étapes (Figure 2). Il faut tout d'abord que la cellule reçoive un signal apoptotique donc la phase de déclenchement ou d'engagement. Ensuite, il y la phase de régulation ou de contrôle puis finalement la phase d'exécution pendant laquelle se déroulerait la cascade enzymatique intracellulaire induisant la mort apoptotique.

### 3.1 Étape de déclenchement de l'apoptose

Une variété de stimulis, autant interne qu'externe, peuvent activer la cellules à devenir apoptotique. Parmi les différents stimulis, on peut énumérer des agents biologiques (récepteurs membranaires, facteurs de transcriptions, les oncoprotéines, infection virale, toxines bactériennes, ...), la suppression de facteurs essentiels à la croissance cellulaire (cytokines, facteurs de croissances et nutritifs, ...), des lésions génomiques de l'ADN (spontanée ou provoquée), l'exposition à des produits chimiques (agents anticancéreux), l'exposition à des agents inducteurs physiques (rayons UV, rayons X, micro-ondes, chaleur, ...). Cette phase se poursuit en de nombreuses modifications biochimiques.

### 3.2 Étape de décision ou d'exécution de l'apoptose

Suite à ces différents stimulis, la cellule reçoit les différents signaux et décide de devenir apoptotique ou pas. Cette étape comprend différents chemin de signaux de transduction entre autre l'activation (ou inactivation) de sérine/thréonine et tyrosine kinases et phosphatases, la synthèse de second messagers, la modification de l'expression de gène et l'activation de protéases spécialisées connu sous le nom de caspases. La décision final pour devenir apoptique dépend de plusieurs facteurs incluant l'équilibre entre les protéines pro-apoptotique et anti-apoptotique (la famille de protéine Bcl-2), l'état métabolique de la cellules et également l'étape du cycle cellulaire dans laquelle la cellule est. Plusieurs arguments suggèrent que le déroulement de cette deuxième étape est contrôlé par la famille des protéines Bcl-2, que l'on retrouve à généralement associées à la membrane externe des mitochondries, du réticulum endoplasmique et du noyau. La famille des protéines Bcl-2 est divisée en deux groupes de protéines, les unes inhibent l'apoptose ( Bcl-2, Bcl-$X_L$, Bcl-w, CED-9,...) et les autres favorise l'apoptose ( Bax, Bid, Bad, Bak, Bcl-$X_s$, ...). Qu'elles soient pro- ou anti-apoptotique, elles ont l'habileté de contrôler le flux ionique entre divers compartiments cellulaires, spécialement entre les mitochondries et le cytoplasme. À cette même étape, il y a activation des caspases sous forme de système d'amplification par auto-activation par elles-mêmes et entre elles mais également par le relâchement de facteurs d'activation de l'apoptose, comme l'AIF (apoptosis-inducing factor) et le cytochrome c, par les mitochondries. L'espace intermembranaire de la mitochondrie comprend plusieurs protéines participant à l'activation de l'apoptose, comme les procaspase-2, -3, -7 et -9, AlF et le cytochrome c. L'activation de ces caspases va mener à un point de non retour puisque par leur activation, elles iront cliver leurs différentes cibles, entre autre , les protéines nécessaires à la survie de la cellule.

### 3.3 Étape de dégradation de l'apoptose

La cellule est maintenant engagée de façon irréversible dans le programme de mort cellulaire qui consiste en la présentation des caractéristiques morphologiques de la signature apoptotique. Donc, par l'activation des différentes caspases, plusieurs protéines nécessaire à la survie de la cellule sont clivées et deviennent non fonctionnelles, comme la polymérase poly(ADN-ribose). D'autres cibles des caspases peuvent êtres activées par celles-ci, entre autre des DNases qui elles-mêmes découperont la chromatine en fragments de haut poids moléculaires.

### 4. Protéines impliquées dans les mécanismes de régulation de l'apoptose

### 4.1.1 La famille des protéines Bcl-2

La protéine Bcl-2 et celles de la même famille sont d'importante modulatrices d'apoptose. Dans cette famille de protéines, il existe deux classes incluant les protéines anti-apoptotiques (Bcl-2, Bcl-$x_L$, Mcl-1, Bcl-w, Bfl-1/A1, Brag-1) et les protéines pro-apoptotiques (Bax, Bak, Bad, Bid, Bik, Bim, Bcl-$x_s$) (Reed, 1996). Les membres de la famille Bcl-2 sont classés selon leur nombre de domaine d'homologie avec Bcl-2 (BH : Bcl-2-homology). La protéine Bcl-2 contient 4 domaines. Toutes les protéines anti-apoptotiques possèdent les quatre domaines, tandis que les protéines pro-apoptotique peuvent être divisées en trois catégories. Un groupe contient les domaines BH1, BH2 et BH3 (Bax, Bak), tandis que l'autre groupe contient seulement le domaine BH3 (Bad, Bid, Bik). Bcl-$x_s$ forme son propre groupe, contenant les domaines BH3 et BH4. Une étude par cristallographique a permis de déterminer la structure de la protéine Bcl-$X_L$. La protéine est donc formée de deux hélice-$\alpha$ centrales entourées de cinq hélice-$\alpha$ « amphipathique ». Il est intéressant de notre que la structure tridimensionnelle est homologue à des toxines bactériennes formant des pores dans des membranes, comme la toxine de la diphtérie et colicin, ce qui pourrait suggérer un mécanisme d'action potentiel pour ces protéines au niveau de la mitochondrie. Une autre caractéristique structurale serait la capacité de ces protéines à s'homo et s'hétéro dimériser les unes avec les autres, par leur domaine BH3, de ce fait, elles pourraient favoriser ou antagoniser leurs fonctions entres elles.

### 4.1.2 Rôles des anti- et pro-apoptotique

### A- Sur la mitochondrie

Tout d'abord, une description des modifications que subit la mitochondrie en situation d'apoptose. Les mitochondries isolées dans une situation d'apoptose subissent ce qui est référé à une transition de perméabilité mitochondriale (MPT). Expérimentalement, la MPT est caractérisée par une augmentation en flèche de la perméabilité de la membrane interne à des particules avec un poids moléculaire de $\leq$ 1500 Da. Cette transition de perméabilité à plusieurs conséquences incluant l'écroulement du $\Delta\psi_m$, enflure osmotique, relâchement du $Ca^{2+}$ matricielle, la création d'espèces oxygèno-réactives, et la rupture de la membrane externe de la mitochondrie menant au relâchement du cytochrome c de l'espace inter-membranaires de la mitochondrie. Une caractérisation biochimique de la mitochondrie a permis d'identifier des pores sensibles au voltage et $Ca^{2+}$ qui contrôle la MPT, qui seront nommés pores PT. Les pores sont localisés à la jonction des membranes interne et externe de la mitochondrie, et ainsi, l'ouverture des pores permettent la communication directe entre la matrice de la mitochondrie et son environnement. Le « voltage-dependant anion channel » (VDAC) et le « adenine nucleotide translocator » (ANT) font partie des pore PT.

Les protéines de la famille Bcl-2 ont différentes distributions cytoplasmique. Les protéines Bcl-2 et Bcl-$x_L$, possèdent une queue hydrophobe en c-terminale contenant une séquence d'insertion de membrane, et la majorité de ces protéines sont reconnues pour être associées aux membranes des mitochondries, de réticulum endoplasmique et à la membrane nucléaire. Dans leur forme inactive, les protéines pro-apoptotiques, Bad, Bax et Bid, ont une location primaire cytoplasmique. Par contre, lors de leur activation, elles se resituent sur les mitochondries. Lorsque Bid est clivé, sa partie terminale COOH se resitue à la surface de la mitochondrie. L'action de ces protéines, soit de prévenir soit d'initier l'apoptose, se situe au niveau des mitochondries, par contre le mécanisme de ces actions demeure controversé et incertain. Il a été démontré qu'en ajoutant la protéine Bax, une protéine pro-apoptotique, à des mitochondries isolées il y a induction du relâchement de cytochrome c, tandis que la sur expression de la protéine Bcl-2 ou Bcl-$X_L$ prévient le relâchement du cytochrome c donc bloque l'apoptose. Il est clair que la famille Bcl-2 est impliquée de façon étroite dans la libération du cytochrome c, la protéine transporteuse d'électron à l'intérieur de la mitochondrie. En plus d'être impliqué dans la phosphorylation oxydative dans la mitochondrie, le cytochrome c est un des constituants (ainsi que la protéine adaptatrice Apaf-1) qui est requis pour l'activation de la caspase-9 dans le cytosol. Comment les membres de la famille Bcl-2 régulent le relâchement du cytochrome c? Plusieurs hypothèses ont été émises, mais aucune n'est prouvée de façon définitive. Il y a trois modèles de base qui peuvent être suggérés.

1- Les membres de la famille Bcl-2 forment un canal qui facilite le transport des protéines. En se basant sur similarité de la structure de Bcl-$X_L$ à la sous-unité de la toxine de la diphtérie qui forme des pores, il a été suggéré que les protéines Bcl-2 pourraient s'insérer dans la membrane externe de la mitochondrie, où elles pourraient former un canal ou bien même un grand trou. Les membres de la famille Bcl-2 en effet peuvent s'insérer dans une couche bi lipidique synthétique, s'oligomériser, et former un canal avec une conduction discrète. Les protéines Bid et Bik peuvent directement induire la mitochondrie à relâcher le cytochrome c sans interagir avec VDAC ou ANT suggérant qu'elles agissent en dehors des pores PT.

2- Les membres de la famille Bcl-2 interagissent avec d'autre protéines pour former des canaux. La famille de protéines Bcl-2 interagie avec plusieurs protéines. Une possibilité serait que les membres de la famille de protéine pro-apoptotique recrute d'autre protéines de la membrane externe de la mitochondrie afin de former un pore assez large pour faire un canal. Une candidate particulièrement intéressante pour une telle protéine serait le canal d'anion voltage dépendant (VDAC), plusieurs membres de la famille Bcl-2 peuvent se lier à elle et réguler son activité de canal. Puisque la grandeur du pore caractérisé du canal VDAC est trop petit pour laisser passer les protéines au travers, ce modèle doit assumer que le VDAC subit un changement conformationnel suite à la liaisons des membres de la famille Bcl-2. Il a été démontré que les protéines Bcl-2 et Bcl-XL favorisent la fermeture des pores PT, tandis que la protéine pro-apoptotique Bax ont l'effet contraire, elle interagit avec ANT et VDAC pour favoriser l'ouverture de ces pores et le re largage du cytochrome c.

3- Les membres de la famille Bcl-2 induit une rupture de la membrane extérieure de la mitochondrie. Il est possible que la famille Bcl-2 contrôle l'homéostasie de la mitochondrie. Dans ce model, le signal apoptotique altèrerais la physiologie de la mitochondrie (par exemple, échange d'ion ou la phosphorylation oxydative) tel que l'organelle gonfle, ce qui donne comme résultat la rupture physique de la membrane externe et le re largage de protéines situées entre les membranes de la mitochondrie, dans le cytosol. Le besoin de former un canal assez gros pour laisser passer le cytochrome c est maintenant plus nécessaire puisque les protéines se diffuseraient simplement par les déchirures dans la double couche lipidique.

Les protéines pro-apoptotiques (Bid) peuvent s'homo dimériser pour former un pore pour laisser sortir le cytochrome c. Les protéines anti-apoptotiques (Bcl-2) ont la capacité de se lier avec des pores PT et ainsi empêcher le re largage des protéines inter-membranaires par contre, les protéines pro-apoptotiques (Bax), vont permettre l'ouverture des pores PT.

La protéine AIF (pour apoptosis-inducing factor) qui a été identifiée et son gène cloné, est capable, à elle seule, d'induire l'apoptose dans des noyaux isolés. Cette molécule est synthétisée dans le cytosol sous forme de précurseur puis est importée dans la mitochondrie. Tout comme le cytochrome c, il s'agit d'une molécule phylogénétiquement ancienne, avec une double fonction : oxydoréduction et facteur apoptogène. Néanmoins, à l'inverse de la voie du cytochrome c, qui nécessite l'activation d'autres facteurs pour induire l'apoptose, la voie d'AIF est au contraire indépendante des caspases et ne nécessite aucun intermédiaire pour provoquer l'apoptose. Elle constituerait entre autres un prototype des voies de l'apoptose indépendantes des caspases.

Les hypothèses concernant les mécanismes d'inhibition de l'apoptose et en particulier la séquestration d'Apaf-1 par Bcl-2 et ses agonistes anti-apoptotiques semblent encore très discutée. Apaf-1 est probablement une cible importante de membres de la famille de Bcl-2, puisque les cellules Apaf-1 déficientes sont réfractaires à divers signaux pro-apoptotiques et qui sont eux-mêmes inhibées par Bcl-2. En plus, une sur-expression d'Apaf-1 a montré que cette protéine était associées avec les protéines de survie comme Bcl-X$_L$ et Bcl-2. En revanche, il a été démontréqu'il n'existe aucune co-immunoprécipitation entre le membres de la famille Bcl-2 et Apaf-1. Apaf-1 a été également trouvée au niveau de sites où les protéines de survie comme Bcl-2 et Bcl-x$_L$ résident tels que les membranes externes de la mitochondrie, l'enveloppe nucléaire et le réticulum endoplasmique.

### 4.1.3 Mécanisme de modulation des protéines de la famille Bcl-2

Plusieurs différents mécanismes existent pour moduler les fonctions des protéines pro- et anti-apoptiques. Tout d'abord, l'état de dimérisation des membres de la famille Bcl-2 affecte leur activité. Une des fonctions des protéines anti-apoptotique Bcl-2 et Bcl-X$_L$ est de se dimériser avec la protéine pro-apoptotiques Bax pour neutraliser son activité. En étant un hétérodimer, Bax est inactif, mais une fois libre de se dimériser avec lui-même, Bax est capable d'induire l'apoptose. Bid, Bik et Bad peuvent agir en inhibant l'action anti-apoptotique de Bcl-2 et Bcl-X$_L$ en formant des hétérodimers. En second lieu, en altérant le niveau d'expression des membres pro- et anti-apoptotique de la famille Bcl-2, peut soit initier l'apoptose soit l'inhiber. Par exemple, lorsque le nombre de Bcl-2 est plus grand ou égale au nombre de Bax, la cellules en question est protégé de l'apoptose. Par contre, lorsque le nombre de Bax dépasse le nombre de Bcl-2, la cellule est plus sujette à devenir apoptotique. En troisième lieu, les protéines de la famille Bcl-2 peuvent être modifiées par phosphorylation. Le meilleur exemple pour ce propos serait la protéine pro-apoptotique Bad. Dans son état non phosphorylée, elle se dimérise avec Bcl-2 et Bcl-X$_L$, neutralisant ainsi leur activité anti-apoptotique. Par contre, lorsque Bad est phosphorylé, elle est séquestrée et de ce fait ne peut interagir et neutraliser Bcl-2 et Bcl- X$_L$. En quatrième lieu, la famille Bcl-2 peut être modifié par clivage. Lors d'une apoptose causé par Fas, il a été démontré que les caspases cliveraient Bcl-2 et Bcl- X$_L$ et que les produits clivés ne sont plus protecteurs et même ils deviennent pro-apoptotique. Bid est une autre protéine de la famille Bcl-2 qui est activé par le clivage des caspases. Tandis que la protéine à sa pleine longueur est inactive, suite au clivage causé par la caspase 8, Bid induit le re largage du cytochrome c par la mitochondrie. Finalement, la conformation des protéines Bcl-2 modifie leur activité. La meilleure évidence pour ce mécanisme viens des études faites sur Bax. Dans son état inactif, Bax existe sous une conformation sous laquelle elle résiste aux clivages protéolytiques. Par contre, suite à son activation et sa re localisation sur la mitochondrie, la région N-terminal de la protéine devient susceptible aux clivages, suggérant qu'il y a bien un changement de conformation qui se produit.

En résumé, les mitochondries ont une place importante dans le déclenchement de l'apoptose. Leur espace inter-membranaire contient plusieurs protéines (cytochrome c, caspase-2, -3, -7, et -9, AIF) lesquelles une fois libérées dans le cytoplasme participent à la phase de dégradation de l'apoptose. L'énigme des mécanismes d'induction et de contrôle de l'apoptose par la mitochondrie repose sur quatre points essentiels : à savoir les molécules de la famille Bcl-2/Bcl-x$_L$ qui pourraient contribuer à la formation de canaux ioniques au niveau des membranes intracellulaires. Les membres pro-apoptotiques de cette famille (comme Bax, Bid, ...) pourraient également intervenir dans la perméabilité des pores PT de la membrane mitochondriale, notamment comme protéines activatrices de l'apoptose. Enfin, les molécules anti-apoptotiques de la famille Bcl-2 pourraient aussi agir en titrant des activateurs endogènes (comme Apaf-1) de l'apoptose. Un dernier point est que certaines pro-caspases ont aussi une localisation mitochondriale. Ainsi, les promoteurs apoptotiques ou les inhibiteurs de la famille Bci-2 régulent l'apoptose grâce à des effets multiples sur les cascades d'activation de caspases, sur le potentiel redox, et sur la fonction de barrière de perméabilité des membranes mitochondriales. Dans l'ensemble ces observation suggèrent donc une implication de la transition de perméabilité dans la régulation de l'apoptose induite par les mitochondries.

## 4.2 Rôle des caspases dans l'apoptose

### 4.2.1 Définition et classification des caspases

Les caspases sont des protéases spécialisées qui sont essentielles pour l'apoptose. Elles sont différentes des autres protéases parce qu'elles utilisent une cystéine pour la catalyse et elles clivent seulement après des résidus d'acide aspartique. Cette spécificité inhabituelle d'avoir un aspartate comme substrat est retrouvé seulement chez une autre protéase, la granzyme B, cependant cette enzyme utilise une sérine comme site actif. Les caspases sont synthétisées comme une chaîne simple de polypeptides et elles sont des zymogènes inactifs. Ces zymogènes sont composés de trois domaines : un pro-domaine N-terminal, et deux autres domaines, p10 et p20, qui sont retrouvés dans l'enzyme mature. Lors de leur activation, chaque chaîne de polypeptide est clivé en deux sous-unité, une grande (p20) et une petite (p10), qui par la suite se dimérisent. Donc, les enzymes matures qui ont été observés, sont des hétéro-tétramères composés de deux hétéro-dimères p20/p10 et de deux sites actifs. Le peptide en N-terminal, est clivé et relâché lors de l'activation. Ce peptide en N-terminal n'est pas requis pour l'activité enzymatique, sont rôle est connu sur caspase 8 et 10, où il agit comme domaine d'interaction avec d'autres protéines pour moduler leur activation. Les caspases 8 et 10 contiennent un « death-effector domain » (DED), alors que les caspase 2 et 9 contiennent un « caspase activation and recruitement domain » (CARD).

Il y a au moins 14 différentes caspases identifiées dans tes tissus de mammifères à ce jour. Il est possible de diviser les caspases -en trois groupes différents par leur spécificité de substrat, c'est à dire, par leur reconnaissance des trois acides aminés qui précèdent l'acide aspartique. Le premier groupe, contient les caspases impliquées dans le processus inflammatoire, donc activation des pro-cytokines, qui inclut les caspases 1, 4 et 5. Ces enzymes sont parfois connues comme les caspases « ICE-like», parce qu'un autre nom pour caspase-1 est « Interleukin-1-converting enzyme» (ICE). Leur motif du tétra-peptide qu'ils reconnaissent et préfère est WEHD, par contre les caspases « ICE-like» sont les plus tolérantes pour ce qui est de la substitution d'acide aminé à comparer les caspases de signalisation et effectrices. Le second groupe de caspases contient les caspases 6, 8, 9 et 10. Ces enzymes sont considérées comme des caspases de signalisation parce qu'elles peuvent activer d'autre caspases et ainsi débuter la cascade. Leur motif de reconnaissance est (LV)EXD. Le dernier groupe contient les caspases 2, 3 et 7. Ces enzymes sont connues sous le nom d'effectrice puisqu'elles clivent plusieurs cibles cellulaires qui donne comme résultat l'apparence morphologique de l'apoptose. L'activation de ces caspases abouti généralement dans un point de « non-retour» dans la mort cellulaire. Les enzymes effectrices sont les plus spécifiques, avec une nécessité d'avoir un acide aspartique dans la première et quatrième position précédent le site de clivage. Elles leur motif de reconnaissance est DEXD. Les caspases les plus récentes, caspases 12-14, n'ont pas encore été assez caractérisées pour pouvoir les classer dans un des trois groupes.

### 4.2.2 Activation des caspases

Il existe trois différents mécanismes pour activer les caspases. Le premier mécanisme est l'activation de la caspase par une autre caspase préalablement activée. La plus part des caspases sont activées suite à un clivage protéolytique du zymogène entre les domaines p20 et p10, et habituellement un autre clivage entre le pro-domaine et le domaine p20. Il est intéressant de noter que tous ces sites de clivages ont lieu après un aspartate, le substrat des caspases, ce qui suggère la possibilité d'une activation par auto catalyse. En effet, la façon la plus facile d'activer une caspase est de la mettre en présence d'une autre caspase déjà activée. Cette stratégie de cascade de caspases est amplement utilisée par la cellule pour l'activation de trois caspases importantes, caspase-3, -6 et -7. Ces trois caspases effectrices sont considérées comme les plus travaillantes de la famille des caspases, et sont habituellement plus nombreuses et actives que les autres.

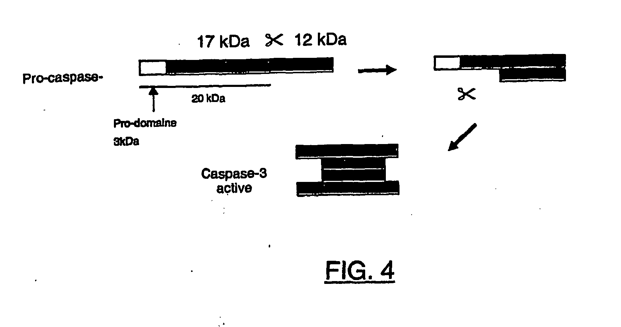

Tel qu'illustré à la Figure 4, le premier clivage se fait entre les domaines p20 et p10 (ici 12 kDa) afin de séparer les deux sous-unités. Le second clivage protéolytique s'effectue entre le pro-domaine et la grande sous-unité et ensuite il y a formation d'un hétérotétramère qui conduit à la caspase mature sous sa forme active.

La cascade de caspases est une méthode très utile pour amplifier le signal pro-apoptotique, mais il ne peut expliquer comment la première, la plus en aval des caspases est activée. Il y a au moins deux autres modèles qui pourraient expliquer l'activation des toutes premières caspases. Le premier est l'induction de l'activation par le rapprochement. Il est connu que la caspase-8 est la caspase initiatrice lors de l'apoptose induit par les récepteurs de mort. Lors de la liaison du ligand à son récepteur, le récepteur de mort CD95/Fas se trimérisent et forment des complexes de signalisation liés à la membrane. Ces complexes recrutent, par des protéines adaptatrices, plusieurs molécules de pro-caspases-8, donnant comme résultat une grande concentration locale de zymogènes. Ce modèle d'activation par rapprochement stipule que sous cette condition de foule, la faible activité protéolytique intrinsèque de la pro-caspase-8 est suffisante pour permettre aux pro-enzymes de se cliver mutuellement et de s'activer les unes les autres. Le dernier modèle d'activation des caspases est l'association de la pro-caspase avec une sous-unité régulatrice. Prenons comme exemple la caspase-9 qui nécessite une association avec des cofacteurs pour son activation. Le cofacteur « apoptotic protease activating factor-1 » (Apaf-1) à été identifié par une approche biochimique comme étant une des deux protéines nécessaires à l'activation de la caspase-9, l'autre étant le cytochrome c. Le complexe que formes ces trois protéines, nécessitant de l'ATP, donne la forme active de la caspase-9 souvent appelé apoptosome. Donc, Apaf-1 n'est pas seulement une protéine activatrice de la caspase-9 mais est une sous-unité essentielle au fonctionnement de celle-ci. En résumé, les caspases effectrices sont généralement activées par des caspases en aval, alors que les caspases initiatrices sont activées par des interactions protéine-protéine régulées.

### 4.2.3 <u>Les victimes des caspases</u>

Les caspases clivent un bon nombre de protéines cellulaires, et le processus de protéolyse est limité puisqu'il y a un petit nombre de coupures qui sont réalisées. Parfois, les clivages entraînent l'activation de la protéine, et d'autre fois, l'inactivation mais jamais la dégradation puisque leur spécificité de substrats distingue les caspases comme étant les endopeptidases les plus strictes. Les caspases clivent plusieurs protéines cellulaires dont le nombre ne cesse d'augmenter. Les protéines de structure, nucléaires et de signalisations sont toutes des cibles des caspases (tableau 1). Il y a différentes protéines du cytosquelette clivées par les caspases comme par exemple, la lamine, $\alpha$-fodrin et l'actine. Le clivage des ces protéines est probablement responsable des changements morphologique observés durant l'apoptose, par exemple le clivage les lamines nucléaire est nécessaire pour le rétrécissement et la bourgeonnement nucléaire. La fragmentation de l'ADN est du à l'activation de la « caspase-activated Dnase » (CAD) par la caspase-3. Cette DNase existe sous forme de complexe inactive avec une sous-unité inhibitrice, ICAD. L'activation de CAD s'effectue donc, par le clivage de la sous-unité inhibitrice par la caspase-3 résultant par le relâchement et l'activation de la sous-unité catalytique.

**Tableau 2- Quelques exemples de victimes des caspases.**

| Catégorie | Cible | Effet |
|---|---|---|
| Signalisation | autres caspases<br>PKC $\delta$<br>Phospholipase $A_2$<br>Bcl-2, Bcl-$x_L$<br>Bid<br>ICAD | Activation<br>Activation, fragmentation nucléaire<br>Activation<br>Formation de fragment pro-apoptotiques<br>Activation<br>Activation de l'endonucléase CAD |
| Nucléaire | Facteur de fragmentation de l'ADN<br>Polymérase poly (ADP-ribose) | Fragmentation de l'ADN<br>Inactivation |
|  | Protéine kinase ADN-dépendante<br>U1 (70 kDa)-snRNP<br>Lamine A et B | Inactivation<br>Diminution de la synthèse des ARNs<br>Désassemblage de la lamine nucléaire |
| Structurale | Actine<br>Gelsoline<br>$\alpha$-Fodrin | Réarrangement du cyto-squelette<br>Réarrangement du cyto-squelette<br>Changement membranaire |

### 4.3 <u>La protéine p53</u>

La protéine p53 est un facteur de transcription qui joue un rôle critique pour la prévention du cancer. La protéine p53 est considérée comme étant le « gardien du génome ». Cette protéine est un bon exemple de comment la décision entre l'apoptose ou la vie peut être faite à un point de vérification activé lorsque l'ADN est endommagée. Tout dépendant du stimulus et la phase dans laquelle la cellule est, l'activation de la p53 peut mener à un arrêt de prolifération cellulaire et à la réparation de l'ADN ou bien à l'apoptose. Alors que le premier stimulus pour activer la p53 est l'ADN endommagée, d'autre stress cellulaire comme la privation de métabolite, un dommage physique, chaleur, et la permet d'oxygène peuvent également activer la p53.

Le niveau de p53 augmente dramatiquement dans les quelques minutes suivant le dommage que la cellule à subit. Cette augmentation est possible par des modifications post-traductionnelles du polypeptide de la p53, sans induction dramatique évidente du niveau ARNm de p53 suite au dommage causé à l'ADN. La modification qui est effectué sur le polypeptide de la p53 suite à un dommage à l'ADN se traduit par la phosphorylation. Dans une cellule ayant subit aucun stress, la protéine p53 possède une demi-vie extrêmement courte mais devient beaucoup plus stable suite à un dommage causé à la cellule. L'instabilité de la protéine p53 dans des conditions normales pour la cellule est relié au fait que p53 est la cible d'une protéolyse induite par de petits peptides, ubiquitines. Donc, p53 se retrouve marqué par les ubiquitines avec l'aide d'une protéine portant le nom de Mdm2, une protéine jouant un rôle dans la régulation négative de p53. Il est démontré que la protéine Mdm2 interagie avec p53 afin qu'elle devienne la cible parfaite des protéases par les ubiquitines. La protéine Mdm2 cause également la translocation de p53 du noyau de la cellule au cytoplasme, où elle subira une protéolyse induite par des ubiquitines. Donc, par la phosphorylation du domaine de régulation dans la partie C-terminale de p53, il est démontré que l'activation de sa liaison à l'ADN selon des séquences spécifique à lieu. De plus, la phosphorylation de la sérine-15 et la sérine-20, en N-terminal de p53, cause l'inhibition de l'interaction entre p53 et Mdm2 par conséquent, augmentent le niveau de p53 et la convertissant en une forme capable d'une activité de transcription. Un grand nombre de kinases phosphorylent p53, incluant la kinase caséine, les kinases reliées aux signaux extra-cellulaire, la protéine kinase C et la kinase Raf-1. Une fois phosphorylé, p53 agit comme étant un facteur de transcription pour augmenter et diminuer la transcription de plusieurs gènes impliqués dans l'apoptose.

Plusieurs protéines régulatrices du cycle cellulaire sont induites par p53, par exemple p21, GADD45 et des membres de la famille 14-3-3. L'habileté qu'a p53 d'induire un arrêt du cycle cellulaire dans la phase $G_1$ suite à un dommage à l'ADN est bien connue et peut s'expliquer par le fait que la protéine p53 une fois stimulée possède une activité de transcription qui permet de transcrire le gène d'inhibition de la kinase dépendante des cyclines (Cdk), la protéine p21. Un nombre élevé de p21 va ensuite inhiber les kinases cycline E/cdk2 et cycline A/cdk2, empêchant ainsi ces kinases de promouvoir la progression du cycle cellulaire. De plus, la protéine p53 est également impliquée dans l'arrêt du cycle cellulaire en phase $G_2$ en partie parce que p53 induit l'expression de la protéine sigma 14-3-3 qui causera la séquestration du complexe cycline B/Cdc2. La protéine p53 peut mener à l'apoptose par l'activation de la transcription de différents gènes donnant des protéines impliquées dans le processus apoptotique. Les protéines qui sont induites sont la protéine Bax, le récepteur Fas et DR5 (récepteur pour le ligand de mort TRAIL), qui sont toutes impliquées dans le processus de l'apoptose. Elle cause également la diminution de l'expression de l'ARNm de Bcl-2, favorisant ainsi l'apoptose. Il semble exister une voie apoptotique induite par p53 qui ne requière pas le relâchement de cytochrome c mais qui nécessite toujours l'activation des caspases. Malgré que l'expression de la protéine bax est augmentée, elle se situe plutôt dans le cytosol et aucune translocation sur la mitochondrie est détectable. Donc, il pourrait exister une autre voie par laquelle la protéine p53 induirait l'apoptose sans qu'il y ait relâchement de cytochrome c.

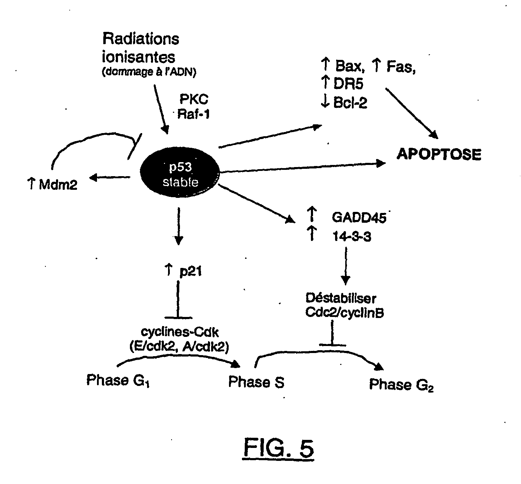

Les résultats finaux suite à un dommage de l'ADN peut être l'arrêt du cycle cellulaire, donc de la croissance, ou bien l'apoptose. Un dommage à l'ADN donne une accumulation et activation de la protéine p53 (Figure 5). Une fois activée, p53 possède une activité de transcription qui augmentera la transcription de différents gènes (GADD45, 14-3-3, Mdm2, p21, Bax, Fas, DR5). Elle peut également réguler à la baisse différents gènes (Bcl-2). En augmentant la p21, qui ira inhiber les kinases cyclines dépendantes (cdk), le cycle cellulaire est alors arrêter en $G_1$. Le cycle cellulaire peut également être arrêté en phase $G_2$ par l'augmentation des protéines GADD45 et 14-3-3 par p53. Le processus d'apoptose est réaliser par différentes protéines (Bax, Fas, DR5) qui sont réguler à la hausse par p53. Une boucle de régulation de la protéine p53 est possible grâce à l'augmentation de Mdm2, une protéine se liant à p53 et favorisant sa dégradation.

## 4.4 Les récepteurs de morts

Les récepteurs de morts sont des récepteurs situés à la surface de la cellule et sont nommés ainsi parce qu'à la liaison avec leur ligand, ils peuvent amorcer le processus d'apoptose. Ces récepteurs, font partis de la famille du récepteur au TNF, en particulier le récepteur TNF-R1 lui-même, le récepteur Fas (aussi appelé CD95 ou Apo-1) et également les récepteurs DR-3, DR-4 et DR-5. Ces récepteurs sont activés par leur ligand qui sont soluble ou membranaire comme le « tumor necrosis factor-$\alpha$ » (TNF-$\alpha$), le Fas-L et le « TNF-related apoptosis inducing ligand » (TRAIL) Les ligands des récepteurs de mort font partis de la famille de cytokine TNF-$\alpha$, et sont des molécules homotrimériques. Des analyses en cristallographie indiquent que chaque monomère du ligand se lie à un récepteur, ce qui indique que la liaison d'un ligand implique la trimérisation de ces récepteurs. L'interaction ligand-récepteur induit la trimérisation du récepteur, qui permet l'association physique de protéines adaptatrices avec les domaines interacting protein death domain » (RIP-DD), favorisent le recrutement et l'activation des caspases proximales comme les pro-caspases-8, -10 et -2, capables alors de transmettre le signal de mort à l'intérieur de la cellule. Prenons comme exemple l'activation du récepteur TNF-R1. Par la liaison de TNFα au récepteur TNF-R1, ce dernier se trimérise donnant comme résultat l'agrégation des domaines de mort, permettant le recrutement de TRADD qui à sont tour recrute une molécule adaptatrice, TRAF2 «TNF receptor-associated factor 2», qui mène à l'activation des voies JNK et NF-κB. TRADD peut également recruter FADD et RIP menant au processus apoptotique et à l'activation de NF-κB, respectivement. RIP peut également recruter RAIDD «RIP-associated ICH-1/CED-3-homologous protein with a death domain» qui par la suite recrute la caspase-2 et induit l'apoptose. Lorsque l'ont prend le récepteur Fas comme modèle d'activation de l'apoptose, le complexe Fas et FADD va recruter la pro-caspase-8 qui formera le complexe qui induira le signal de mort, « death-inducing signal complex » (DISC). Une fois assemblé, le DISC va causer une auto-activation rapide de la caspase-8 qui ira activer la caspase-3 et causera l'apoptose de la cellule. Ainsi, cette première voie d'action du récepteur Fas est une voie rapide court-circuitant la mitochondrie et qui ne nécessite pas l'apport de nouvelles molécules car elle est basée sur l'interaction de molécules préexistantes.

Toutefois, il a été récemment montré que l'activation de la caspase-8, suite à la trimérisation du récepteur Fas, pouvait provoquer également le clivage de Bid, une protéine pro-apoptotique de la famille Bcl-2. Ce clivage entraîne la pénétration d'une forme tronquée de Bid dans la mitochondrie avec comme conséquence, la sortie du cytochrome c, puis la dépolarisation du potentiel de membrane mitochondriale et l'apoptose. Il est également démontré que lorsqu'il y a activation de DR-4 et DR5 par TRAIL, la caspase-8 est activé. La voie utilisé n'est pas encore bien élucidée mais plusieurs confirment qu'il y a activation des caspases-3 et -9 suite à l'activation de la caspase-8 et observent que Bid est clivé suite à l'activation de la caspase-8. Il est possible que par la liaison de TRAIL aux récepteurs DR4 ou DR5, il y a activation de la caspase-8, par recrutement à l'aide d'une molécule adaptatrice, qui à la fois active la caspase-3 et clive Bid, afin que ce dernier devienne actif et permettre le re largage de cytochrome c qui aura comme effet d'activer la caspase-9 qui à sont tour activera la caspases-3 ce qui causera une amplification de la cascade des caspases et donnera la mort à la cellule.

En résumé, il y aurait donc au moins deux voies de transduction du signal apoptotique par certains récepteurs de mort, l'une directe rapide, l'autre plus lente mettant en jeu le relais mitochondriale. C'est pourquoi certains auteurs classent les cellules (type I ou II) selon leur mode d'induction de l'apoptose par Fas. Comme par exemple, l'activation de Fas, dans certaines cellules, mène quasiment exclusivement à la voie des caspases seulement (cellules de type I). Ces cellules ne démontre habituellement aucune implication de la part des mitochondries, et la mort cellulaire n'est pas inhibée par Bcl-2 ou Bcl-x$_L$. Dans d'autres cellules, l'activation de Fas enclenche la voie utilisant les mitochondries en grande partie, et ce suite à l'activation de la caspase-8. Les protéines anti-apoptotique Bcl-2 ou Bcl-x$_L$ peuvent inhiber l'apoptose seulement dans les cellules de type II, par leur action de prévenir le relâchement de cytochrome c dans le cytosol.

Plusieurs stimulus peuvent initier l'apoptose, par contre des altérations morphologiques et biochimiques courantes sont observées et ce indépendamment du stimulus initiale. Les recherches suggèrent que la plupart des signaux apoptotiques converge sur un nombre limité de voies menant à l'apoptose.

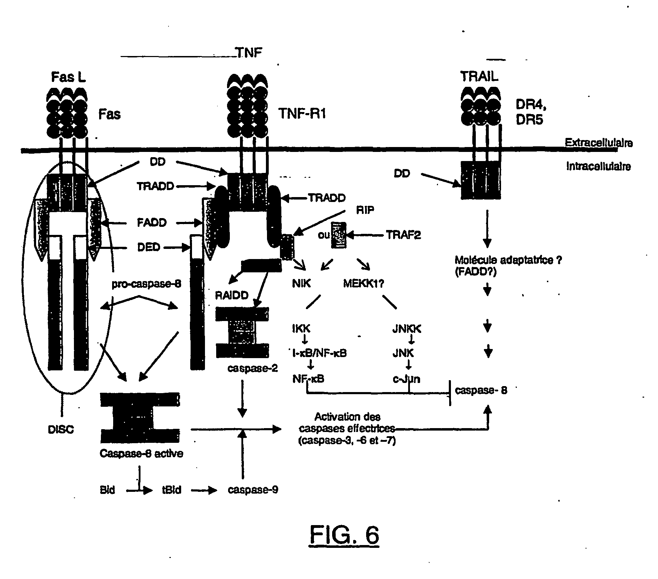

La Figure 6 montre la structure des membres des récepteurs de morts membranaire et leur interactions avec les principaux effecteurs cytoplasmiques impliqués dans les voies apoptotiques. Les flèches rouges indique une activation directe de la caspase-8, et les flèches noires une inhibition de l'apoptose avec des étapes intermédiaires (activation de kinases/facteur de transcription). Les abréviations mentonnées dans cette figure ont la signification suivante: DD, death domain; TRADD, TNF-receptor associated death domain; FADD, Fas-associated death domain, DISC, death-inducing signaling complex; RIP, receptor-interacting protein, TRAF2, TNF-receptor associated factor-2; NF-κB, nuclear factor kappa B, I-κB, inhibitory kappa B; JNKK, JNK kinase; TNF, tumor necrosis factor; TNFR, récepteur du TNF; Fas L, Fas ligand; TRAIL, tumor necrosis factor-related apoptosis inducing ligand; DR4-5, death receptor 4-5; DED, death-effector domain; RAIDD, RIP-associated ICH-1/CED-3-homologous protein with a death domain;

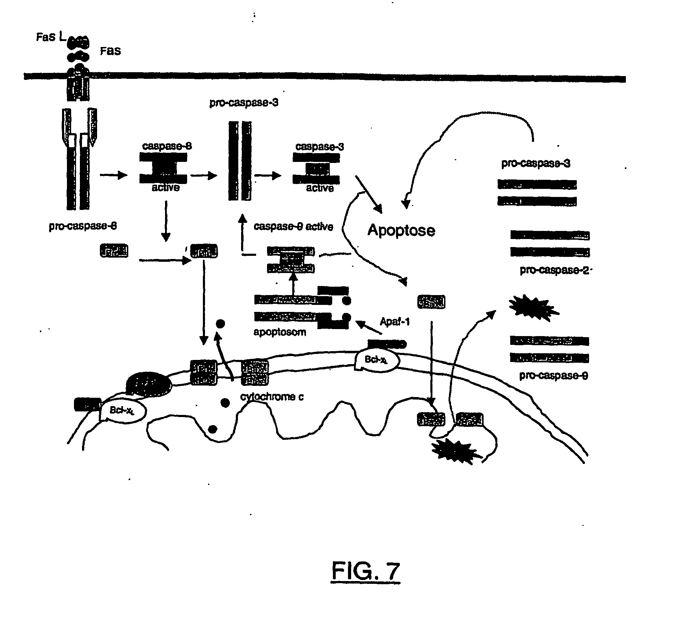

Le processus d'apoptose requiert la participation de plusieurs voies afin d'activer les caspases (Figure 7). Les deux plus connues et les mieux caractérisées sont la transduction du signal apoptotique par les récepteurs de mort et l'autre voie plus interne à la cellule est l'apoptose induite par les changements de l'intégrité mitochondriale, en particulier la libération de facteurs apoptogènes comme le cytochrome c et AIF. Il existe des interconnexions entre ces deux voies de signalisation et des boucles d'amplification du signal. Abréviations: AIF, apoptosis-inducing factor; tBid, protéine tronquée.

### 4.5 La protéine kinase C et *Nurr77*

Les protéines kinase C (PKC) font parties d'une famille de sérine/thréonine kinase. Il y a au moins 11 différentes iso-enzymes de PKC que l'on peut diviser en trois sous-groupe, en se basant sur leur structure et leur mécanisme de réponse à des facteurs de régulation. Les PKC conventionnelles (α, βI, βII, γ) sont dépendantes du Ca$^{2+}$ et sont activées par le diacylglycérole (DAG) ou par le 12-o-tetradecanoylphorbol-3-acetate (PMA) de façon *in vivo*. Le second sous-groupe (δ, ε, η, θ, μ), les iso-types nouveaux, ne répond au Ca$^{2+}$ mais il est activé par le DAG et PMA. Le dernier sous-groupe (λ, ζ, τ), les PKC atypiques, sont insensibles autant au Ca$^{2+}$ qu'au DAG. Les PKC sont responsable pour la transduction de plusieurs signaux cellulaire lors d'une variété de processus cellulaire comme la croissance cellulaire, la différenciation, la transformation maligne et l'apoptose. Les PKC sont également reconnues pour moduler l'activité de différentes protéines de membranes comme les protéines transporteuses, les canaux, et les protéines de membranes reliées au cytosquelette. Puisqu'elles possèdent différents rôles, on constate que leur activation peut donner différents résultats qui peuvent même être des résultats opposés. Il a été démontré que l'activation de la PKC induit l'apoptose sur une lignée de cellules cancéreuses gastriques traitées avec du PMA et ce par l'intermédiaire de la caspase-3 et de protéases sérines. Il a également été démontré que la PKC inhibe l'apoptose induite par le récepteur Fas et ce par la modulation de la perte de potassium ($K^+$) et l'inhibition de l'activité des caspase-8 et -3. Il est démontré par différent chercheur que les PKC ont un rôle dans la régulation transcriptronnelle de l'expression des gènes Fas et FasL. L'équipe de Park à démontré que l'habileté des PKC à induire l'expression de Fas est possible grâce au gène TDAG51 (T-cell death-associated gene) dans les cellules ayant le type sauvage seulement. Il existe un autre médiateur dans l'expression du système Fas/FasL qui est un membre de la superfamille des récepteurs stéroïdiens nucléaires orphelins, Nurr77. Nurr77 joue un rôle dans la croissance cellulaire par son rôle de facteur de transcription nucléaire. Il a été démontré que par l'ajout de Nurr77 sous forme transgénique dans des thymocytes, une augmentation de Fas ligand est obtenue ce qui suggère que Nurr77 peut mener la cellules a devenir apoptotique par l'induction de l'expression de Fas ligand. Un autre rôle fut identifié pour Nurr77. Nurr77 serait capable de réguler l'apoptose par un moyen indépendant de son activité de régulation transcriptionnelle, entre autre par sa re-localisation du noyau vers les mitochondries causant le relâchement de cytochrome c. Donc, par son rôle de facteur de transcription et son rôle de protéine pouvant causer le relâchement de cytochrome c, Nurr77 peut ainsi amener une cellule à devenir apoptotique.

### 5 Le cancer du côlon et ses moyens de défense

À l'intérieure d'un tissu l'homéostasie est maintenue par la balance entre la croissance cellulaire et la mort cellulaire programmée. Une cellule cancéreuse peut être définie comme une cellule qui a survécue au processus d'apoptose et fait maintenant partie des cellules pouvant contribuer à la formation de tumeur. Plusieurs causes peuvent mener à la formation de cancer, autant une faille dans le processus de croissance qu'une faille dans le processus d'apoptose. Ces failles sont responsables de nombreuses maladies y compris le cancer. Une accumulation de cellule peut se produire lorsque le taux de mort est normal mais que le taux de croissance est anormalement élevé ou bien lorsque le taux de croissance est normal mais que le taux de mortalité est anormalement bas. La transformation maligne et la progression tumorale sont des processus complexe nécessitant un bon nombre d'altération génétique.

Normalement, les cellules tumorales sont éliminées par présentation, à la surface membranaire, d'antigènes aux lymphocytes T cytotoxiques (CTL) et aux «natural killer» (NK) par le complexe majeur d'histocompatibilité de classe I (CMH I) à leur surface ce qui permet d'activer la réponse immunitaire. Les cellules cytotoxiques peuvent reconnaître les cellules tumorales par l'expression, à leur surface, d'antigènes viraux (non-soi), d'antigènes-néo (issus de protéine du soi muté), d'antigènes du soi non-muté mais sur-exprimés, d'antigènes oncofoetales (gène éteint au cours de l'embryogenèse qui serait spontanément re-transcrit).

Parfois, les cellules tumorales développent des alternatives afin d'échapper au système immunitaire. Plusieurs mécanismes sont créés par la cellule tumorale, certains causant une aberration de la présentation d'antigène à la surface de la cellule cancéreuse: La diminution de l'expression du CMH I (cellule tumorale n'est plus reconnaissable par CTL, mais peut être détruite par NK) et l'altérations de la structure du CMH I (reconnaissance ni de CTL ni de NK). Ensuite, ceux causant une mauvaise interaction entre cellules cytotoxiques et la cellule cancéreuse : la diminution de la sécrétion de molécules co-stimulatrices ou d'adhésions (essentiel dans la présentation antigénique, peut causer l'anergie), la diminution ou mutation du récepteur Fas membranaire et du récepteur DR4 ou DR5 (cellule tumorale moins sensible à l'attaque des CTL ou NK). Les cellules tumorales peuvent également sécréter des cytokines qui favorisent leur croissance. Une étude démontre que certain cancer du côlon, que la production d'IL-10 (qui a pour effet d'inhiber la production de cellule T CD4$^+$ de type Th1 et d'inhiber les fonctions des macrophages) est régulé par la production local de cytokines pro-inflammatoire comme IL-6 et IFN-$\gamma$. Une seconde étude faites sur 9 lignées cellulaires de cancer du côlon, démontre que celles-ci produisent des facteurs immunosuppresseur, inhibant la prolifération des cellules T.

Le concept d'immunité cellulaire est basé sur la capacité des lymphocytes T cytotoxiques à éliminer les cellules tumorales. Le mode d'action des CTL est d'induire l'apoptose chez les cellules cancéreuses par deux mécanismes : par les récepteurs de morts et par la voie perforine/granzyme B. Par contre, dans certain cas, les cellules tumorales développent des moyens pour contre-attaquer la surveillance du système immunitaire et font en sorte que le processus d'apoptose ne soit pas déclenché. Des équipes de chercheurs ont démontré que les cellules cancéreuses du côlon peuvent se défendre contre les CTL par l'expression de Fas ligand à leur surface membranaire, et ce en causant la mort des CTL par la liaison du récepteur Fas des CTL et le ligand Fas des cellules cancéreuses. Par contre, des résultats contradictoires ont démentis cette hypothèse. Donc, on peut constater que cette hypothèse reste un sujet de recherche assez controversé. Un autre mécanisme peut être développé par les cellules cancéreuses afin d'éviter l'apoptose et de neutraliser les CTL : la sécrétion sous forme soluble du ligand Fas. Donc, dans ce scénario, la cellule cancéreuse sécrète le ligand Fas sous forme soluble, en causant une mutation dans le domaine trans-membranaire de la protéine donc celle-ci ne possède plus de point d'encrage à la membrane cellulaire, et ainsi le ligand Fas se lie à son ligand situé à la surface du CTL et causant donc l'apoptose chez le CTL. Il est démontré que dans certain cas, les CTL peuvent induire l'apoptose chez les cellules cancéreuses exprimant le ligand Fas par le mécanisme de perforine/granzyme B. Par contre, dans d'autre cas les cellules tumorales ont su développer un mécanisme contrecarrant l'efficacité du mécanisme perforine/granzyme B par la sur-expression d'un inhibiteur de sérine protéase, PI-9.

Les cellules tumorales peuvent développer plein de mécanismes pour éviter la mort mais parfois ces mécanismes peuvent amener la cellule à s'auto-suicider ou bien à causer la mort fratricide (mort d'une cellule cancéreuse voisine). Par exemple, si la cellule se met à sécréter le ligand Fas à sa surface ou sous forme soluble, celui-ci pourrait aller se lier au récepteur Fas d'une cellule voisine ou même sur un récepteur Fas situé à la surface de la même cellule.

Il existe d'autres façon qu'une cellule cancéreuse peut échapper à l'apoptose entre autre par la mutation de certaine protéine impliquer dans le processus d'apoptose comme par exemple la protéine p53. Cette protéine est une des cibles de la plus part des cancers du côlon. Donc par la mutation, ce qui la prive de ces fonctions de gardien du génome et d'activation du processus d'apoptose, même si la cellule est endommagée au niveau de son ADN par différents traitements (radiothérapie et/ou chimiothérapie) elle risque quand même d'échapper à l'apoptose. Il y a également la sur-expression de la protéine c-FLIP, cette protéine inhibe l'étape de liaison entre les protéines recruteuses FADD et la caspase-8 par sa liaison à FADD. Il est démontré que la sur-expression de cette protéine est fréquente dans différents cancer du côlon ce qui pourrait contribuer à la transformation tumorale *in vivo*.

Naturellement, la liste des mécanismes d'évasion peut-être longue car les cellules cancéreuses semble développer des stratégies leur permettant de contoumer plusieurs obstacles qu'on ne cesse de découvrir.

### 5.1 Thérapies contre le cancer du côlon

Les traitements contre le cancer du côlon obtiennent un haut taux de réussite lorsqu'il est localisé dans le côlon et qu'il n'a pas traversé les parois du côlon. On attribue ce haut taux de réussite au diagnostique obtenu tôt dans le développement de la maladie. Selon statistique Canada, le cancer du côlon est le troisième cancer causant le plus de mort au Canada par année, 6500 morts pour l'année 2000 et 17 000 nouveaux cas. À cette fin, plusieurs traitements sont maintenant disponible afin de contre carrer cette maladie.

### 5.1.1 Stades du cancer du côlon selon Dukes

Les traitements sont donnés selon le stade de la maladie, il existe deux systèmes afin de classifier dans quel stade le cancer de côlon d'un patient se situe : la classification de Duke et le système TNM (« tumor characteristics, nodal involvelment and amount of metastasis »). La classification de Duke est la plus utilisé à ce jour donc en voici un résumé: Le stade A représente l'étape où le cancer du côlon est limité à la muqueuse ou à la sous-muqueuse du côlon. Les options de traitement à ce stade sont soit la colosectomie, lors d'une lésion superficielle causée par le cancer, soit une excision de la portion affecté, lors d'une lésion plus profonde. Le taux de survie post-opératoire est de 90%. Le stade B se mesure selon de degré d'envahissement d'organes ou de tissus situés à proximité de la tumeur. Le traitement à donner dans ces cas est naturellement l'excision de la tumeur et considération pour l'utilisation de la chimiothérapie et/ou radiothérapie. Le taux de survie se situe entre 70-80%. Le stade C implique l'envahissement de nodules lymphatiques et la formation de métastases dans les vaisseaux sanguins majeurs. Les traitements à prescrire sont l'excision des parties malades, la chimiothérapie avec la combinaison d'adjuvant. Le stade D, le dernier, des métastases distantes sont présentes. Les traitements sont l'ablation des différentes métastases isolées (foie, poumon, ovaires), ainsi que de la chimiothérapie ou/et radiothérapie palliative. Le taux de survie suite à l'opération est généralement moins de 5 ans.

### 5.1.2 Chimiothérapie

Le médicament le plus utilisé pour la chimiothérapie est le 5-fluoro-uracil (5FU), il est administré sous forme intra-veineuse. Des études ont démontré que l'utilisation du 5FU, suite à l'excision de la tumeur, est plus bénéfique pour les patients en phase C de Dukes que l'excision seul. Ensuite, le désir d'obtenir de meilleurs résultats pour vaincre le cancer du côlon, les traitements combinés sont prescrits à différents patients atteints de cancer du côlon en phase B et C. Plusieurs études démontrent que par la combinaison du 5FU avec un adjuvant, levamisole ou leucovorine, de meilleurs résultats sont obtenus pour le stage C de Dukes. Le levamisole est reconnu pour améliorer l'efficacité du 5FU, par cette combinaison, il est démontré que les cas de récurrences sont diminués, et le mécanisme est probablement relié par l'activation de macrophage qui détruisent les cellules tumorales restantes puisqu'il semble moduler le système immunitaire positivement. La leucovorine est un acide folique qui est donné pour éviter ies effets négatifs hématologiques, donc aide à garder les cellules saines en vie et laissant les cellules cancéreuses sujettes à l'action cytotoxique du 5FU. En générale, les études s'entendent pour dire que les traitements avec la combinaison d'adjuvant a un effet positif sur le taux de survie ainsi que le temps de récurrence de la tumeur suite à l'ablation du cancer en comparaison avec l'utilisation du 5FU seule. Le mécanisme d'action du 5FU est de se lier à une enzyme à l'intérieur de la cellule, qui permet la synthèse de la thymine lors de la réplication de l'ADN, et l'inhiber. Par conséquent, la cellule incapable de se diviser va mourir. D'autres médicaments peuvent agir contre le cancer du côlon et sont présentement en étude clinique, ou le seront prochainement. Les différents médicaments sont les suivants : l'Irinotecan (Camptosar, CPT-11), l'Oxaliplatine et le Ralitrexed, Xeloda (Capecitabine). L'Irinotecan agit en inhibant la topoïsomérase I, nécessaire pour donner une certaine forme à l'ADN lors de la translation, transcription, et la réplication. L'Oxaliplatine, agit sur l'ADN en formant des ponts dans l'ADN inhibant ainsi sa synthèse et sa réplication. Le Ralitrexed à un rôle semblable au 5FU puisqu'il interfère dans la phase de synthèse de l'ADN en bloquant l'enzyme synthétisant la thymine. Le Xeloda, est un comprimé oral qui se transforme en 5FU suite à l'ingestion.

### 5.1.3 Mécanisme de destruction des cellules cancéreuses par chimiothérapie

Lorsque l'effet du médicament sur les cellules est connu, il est possible de comprendre le mécanisme qui amène la cellule à devenir apoptotique. Le 5FU étant un inhibiteur de la thymine synthétase, celui-ci cause un dommage à l'ADN lors de la division cellulaire en la privant d'une des quatre bases pyrimidiques constituant l'ADN. Par ce dommage causé à l'ADN, le 5FU est responsable de l'activation de la protéine gardienne du génome, la p53. Il est démontré que cette protéine, par sont activité de transcription, peut moduler l'expression de différentes protéines impliquées dans le processus d'apoptose comme par exemple la protéine Bax, Bak et Bcl-2. Donc, lorsque l'expression de Bax est augmenté et que l'expression de Bcl-2 est diminuer, les chances que le potentiel membranaire mitochondriale soit perdu est augmenté ce qui amène à enclencher le processus d'apoptose par la voie mitochondriale. Ces protéines étant régulées par la protéine p53, détermine la sensibilité de la cellule à la chimiothérapie puisque dans la majorité des cancers du côlon, p53 est mutée. Malgré que p53 est mutée, il est possible d'observer la mort de ces cellules suite à l'ajout de 5FU.

Il est également démontré que le traitement des cellules cancéreuses au 5FU induit l'expression du récepteur Fas et du ligand Fas à la surface des cellules cancéreuses. L'induction de l'expression du récepteur Fas permet donc une meilleure chance d'élimination de la cellule cancéreuse par les cellules immunitaires. Il est également proposé que par l'induction de l'expression du récepteur Fas et du ligand Fas à la surface des cellules cancéreuses traitées, une mort autocrine, paracrine ou fratricide peut s'en suivre. Puisque les cellules expriment le récepteur et le ligand, il pourrait y avoir une liaison croisée entre le récepteur et le ligand d'une même cellule (autocrine),ou le récepteur d'une cellule et le ligand d'une autre cellule (paracrine). Différentes équipes de chercheurs ont démontré que l'apoptose induite par le traitement de chimiothérapie, implique l'activation des caspase-3 et -8 que ce soit une cellule de type I ou II. Il est suggéré que dans les cellules de type I, les deux voies d'initiation d'apoptose sont emprunté lors de la chimiothérapie. Donc, le traitement favorise l'agrégation du récepteur Fas ce qui cause l'activation de la caspase-8 qui ira activé directement la caspase-3, et la caspase-8 peut également activer la protéine Bid par clivage qui ira activer la voies mitochondriale de l'apoptose. Pour ce qui est des cellules de type II, l'apoptose est contrôlée par la voie mitochondriale puisque l'utilisation d'inhibiteur des FADD n'a pas diminuer l'apoptose causé par la chimiothérapie dans ce type de cellule. Donc, l'activation de la caspase-8 dans les cellules de type II a lieu suite aux événements de signalisation de la mitochondrie.

D'autres études ont démontré que le dommage causé à l'ADN par chimiothérapie ou par irradiation, augmente l'expression du récepteur de mort DR5 de façon dépendant et indépendante de p53.

On observe l'implication de plusieurs protéines suite à un traitement de chimiothérapie. Il est important de bien comprendre les mécanismes utilisés par ce traitement puisque plusieurs cancers développent différentes astuces pour éviter la mort.

### 6 Les bactéries lactiques

C'est le scientifique E. Metchnikoff (1845-1919) qui a proposé que la longévité et la santé du peuple bulgare est attribuable à leur ingestion de produit laitier fermenté. Il était bien connu que certaine bactéries sont pathogènes pour l'organisme, donc il fut proposé que l'on substitut ces bactéries par des bactéries du yogourt puisqu'elles étaient depuis longtemps utilisé sans aucunes craintes. Plusieurs caractéristiques existe afin de définir les bonnes bactéries lactiques : elles doivent garder leur activité et leur viabilité avant leur consommation, elles doivent survivre le tractus gastro-intestinal, elles doivent être capable de survivre et de croître dans les intestins et éventuellement produire des effets bénéfiques. De plus, les micro-organismes ne doivent pas être ni pathologiques ni toxiques.

Depuis, plusieurs tentatives ont été faites afin d'améliorer la santé par modification de la flore intestinal par des bactéries lactiques vivantes. Aujourd'hui, les effets bénéfiques de ces bactéries lactiques sont bien identifiés et ont tente maintenant d'expliquer le ou les mécanisme(s) relié(s) à ces bienfaits. L'équipe de Salminen a résumé les effets bénéfiques les plus importants, soutenus par des preuves scientifiques, tel que la modulation immunitaire et le renforcement de la barrière mucosale des intestins. D'autre équipes ont démontré, dans la souris, que la croissance ainsi que les métastase de cancer peuvent être inhibé par la souche de *lactobacillus casei*. Différents mécanismes sont proposés afin d'expliquer à quoi serait du ces bienfaits : la modification de la flore intestinale, adhérence à la muqueuse intestinale avec la capacité de prévenir l'adhérence de bactéries pathogènes ou l'activation de pathogènes, la modification des protéines alimentaire par la microflore intestinale, la modification de la capacité enzymatique bactérienne, spécialement celles suggérées pour être reliées à l'induction de cancer, et finalement l'influence sur la perméabilité de la muqueuse intestinale.

La plus part des études indiquent un potentiel thérapeutique des bactéries lactiques et du yogourt qui est dû principalement par le changement de la micro-écologie gastro-intestinal. L'efficacité des bactéries lactiques est accrue par leur capacité d'adhérence à la paroi intestinale puisque les souches adhérentes ont un avantage compétitif, important pour maintenir leur place dans le tractus gastro-intestinal. Par contre, aucunes souches n'a encore été démontrée pour adhérée de façon permanente. En augmentant la quantité de bactéries lactiques dans les intestins, il est possible de supprimer la croissance de bactéries pathogènes, qui contribuent en retour à une réduction des infections. Un épithélium intestinal intact avec une flore intestinale optimale représente une barrière contre les invasions ou la colonisation par des micro-organismes pathogènes, des antigènes et des composés néfastes pour le tractus intestinal.

En général, la consommation de bactéries lactiques agit par un renforcement de la réponse immunitaire non-spécifique ou agit comme adjuvant dans la réponse immune antigène-spécifique. Des études sur des animaux ont démontrées que le tissu lymphoïde associé aux intestins, est stimulé par des bactéries lactiques vivantes, résultant en une production de cytokines et d'anticorps (IgA) et une augmentation de l'activité mitogénique des cellules formant les plaques de Peyer et des splénocytes. Dans les études sur des cellules humaines, la production de cytokine, l'activité phagocytaire, la production d'anticorps, les fonctions des cellules T et l'activité des cellules NK, sont augmentés par la consommation de yogourt ou lorsque les cellules sont exposées aux bactéries lactiques de façon in vitro.

Certaines preuves indiquent que le yogourt stimulant le système immunitaire peut être associé avec la diminution d'incidence pathologiques comme le cancer, les désordres gastro-intestinal et les symptôme d'allergies.

### 6.1 Propriétés anti-cancéreuse

Les bactéries lactiques auraient des propriétés antinéoplasiques dans une variété de lignée cellulaires cancéreuses d'origine humaine et animal. En bref, les bactéries lactiques réduisent la viabilité des cellules tumorales, diminuent la carcinogenèse induite dans le côlon et le foie, inhibe l'activité mutagénique et se lie à des composés potentiellement mutagéniques. Malgré qu'aucun mécanisme n'est connu, il est suggéré que l'inactivation ou l'inhibition de la formation de cancer dans le tractus intestinal est induite.

Il existe des intérêts considérables pour l'activité métaboliques de la microflore intestinal, spécialement en relation avec l'étiologie du cancer du côlon. Les études ont portées entre autre sur la mesure d'enzymes clés: β-glucuronidase, azo-réductase et nitro-réductase. Ces enzymes catalysent la conversion de carcinogènes indirecte en carcinogène dans les intestins. Par l'absorption de bactéries lactiques, celles-ci diminueraient l'activité de ces enzymes clées et ainsi préviendrait la formation de tumeurs. Un supplément oral de bactéries lactiques *(L. acidophilus)* d'origine humaine cause une diminution significative des ces trois enzymes clées. Ces résultats ont été partiellement confirmé par l'équipe de Marteau qui a enregistré une diminution seulement de la nitro-réductase chez des 9 sujets qui ont ingéré des bactéries lactiques (*L. acidophilus, B. bifidum*) pendant 3 semaines. Les recherches se sont continuées sur un modele de cancer du côlon animal induit chimiquement par le 1,2-diméthylhydrazine dihydrochloride (DMH). L'activation du DMH se fait dans le grand intestin et ces l'enzyme bactérienne β-glucuronidase qui le transforme en une carcinogène potentiel. La suppression de cette enzyme peut réduire l'activation du DMH et par subséquent la formation du tumeur. Ces études démontrent que l'addition de bactéries lactiques peut retarder la formation du cancer du côlon en prolongeant l'induction, indiquant que les *lactobacillus* peuvent ralentir le développement de la tumeur dans le modèle expérimental animal.

En résumé, plusieurs conclusions sont suggérés en ce qui a attrait aux fonctions inhibitrices des bactéries lactiques envers le cancer du côlon. Entre autre, l'augmentation ou la stimulation des fonctions immunitaires, pourrait contribuer à diminuer le risque du développement ou la réapparition du cancer. Également, les bactéries lactiques pourraient prendre la place des bactéries pathogènes qui seraient à l'origine de la formation de composés mutagènes causant le cancer du côlon. Il existe donc un besoin de trouver des méthodes plus efficaces ou ayant moins d'effets secondaires que les traitements déjà disponibles pour traiter la maladie.

### SOMMAIRE DE L'INVENTION

Tel que mentionné ci-haut, de nouvelles propriétés de ces bactéries ont été découvertes. En effet, il a été découvert de façon surprenante que l'effet des bactéries lactiques, notamment celles contenues dans le produit vendu sous la marque de commerce de Bio- K + International, en combinaison avec un agent anticancéreux puissent causer l'apoptose cellulaire.

Plusieurs mécanismes peuvent être à l'origine d'un tel phénomène. Par exemple, les bactéries lactiques peuvent éviter la mutation qui sont à l'origine des cancers. Elles peuvent également prévenir la progression des tumeurs en renforçant le système immunitaire.

La demanderesse a découvert de façon surprenante que la consommation de bactéries lactiques aurait pour effet de prévenir la formartion d'un cancer, notamment le cancer du colon.

La demanderesse a également découvert de façon surprenante que l'utilisation de bactéries lactiques en combinaison avec un agent anticancéreux ont pour effet d'augmenter la susceptibilité des cellules cancéreuses à l'apoptose.

Ainsi, la présente invention concerne l'utilisation d'une souche bactérienne lactique pour renforcer une réponse immunitaire chez un mammifère dans le but de prévenir ou traiter un cancer, ladite souche étant Lactobacillus acidophilus I-1492 déposée à la CNCM.

Un autre objet est l'utilisation d'une souche bactérienne lactique pour faciliter l'induction de l'apoptose des cellules d'un cancer, ladite souche étant Lactobacillus acidophilus I-1492 déposée à la CNCM.

Un autre objet est l'utilisation d'une souche bactérienne lactique pour la fabrication d'un médicament destiné au traitement ou la prévention du cancer, ladite souche étant Lactobacillus acidophilus I-1492 déposée à la CNCM.

Selon un mode de réalisation préféré, la souche bactérienne est sous une forme vivante ou irradiée.

Un autre objet de la présente invention est de fournir une composition pour traiter ou prévenir un cancer, tel le cancer du colon. La composition de la présente invention comprend une quantité efficace d'une souche bactérienne lactique telle que définie précédemment et un véhicule pharmaceutiquement acceptable. Selon un mode de réalisation préféré de l'invention, la composition comprend également un agent anticancéreux, tel le 5 fluoro-uracil.

Un autre objet de la présente invention vise une méthode pour faciliter l'apoptose des cellules d'un cancer chez un mammifère, caractérisée en ce qu'elle comprend l'administration chez ledit mammifère d'une composition tel que définie précédemment.

Un autre objet de la présente invention concerne l'utilisation de bactéries lactiques pour augmenter l'apoptose de cellule cancéreuse, lesdites bactéries étant Lactobacillus acidophilus I-1492 déposée à la CNCM.

Un autre objet de la présente invention concerne l'utilisation en combinaison de bactéries lactiques et d'un agent anticancéreux, tel que le 5 FU pour le traitement d'un cancer du colon, lesdites bactéries étant Lactobacillus acidophilus I-1492 déposée à la CNCM.

Un autre objet de l'invention est de fournir un kit pour prévenir ou traiter un cancer chez un mammifère, caractérisée en ce qu'il comprend un récipient contenant une composition tel que définie précédemment.

## BRÈVE DESCRIPTION DES DESSINS

Figure 1 est un schéma illustrant une analyse typique du pourcentage d'apoptose par cytométrie en flux.

Figure 2 est un schéma illustrant un modèle de processus apoptique.

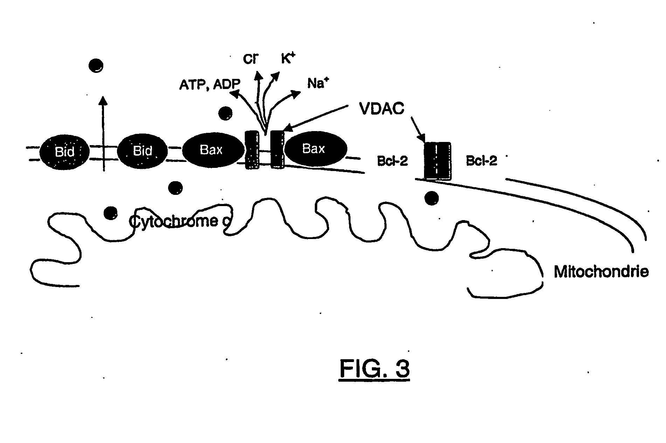

Figure 3 esr un schéma illustrant des hypothèses du relâchement par la famille de protéines Bcl-2.

Figure 4 est un schéma illustrant la maturation protéolytique de la pro-caspase-3.

Figure 5 est un schéma illustrant la récapitulation des effets de l'activation de p53.

Figure 6 est un schéma illustrant la structure des membres des récepteurs de morts membranaires et leur interactions avec les principaux effecteurs cutoplasmiques impliqués dans les voies apoptotiques.

Figure 7 est un schéma illustrant les interconnections entre deux différentes voies de transductions de l'apoptose.

Figure 8 est un graphique montrant la dose de 5-fluoro-uracil optimale pour obtenir 50% de mortalité cellulaire.

Figure 9 montre une présentation visuelle de schémas d'apoptose obtenus par cytométrie en flux.

Figue 10 est un graphique montrant l'effet de la compostion selon un mode de réalisation préféré de l'invention sur la viabilité des cellules LS 513.

Figure 11 est un graphique montrant l'effet de la compostion selon un mode de réalisation préférée de l'invention sur l'apoptose des cellules LS 513.

Figures 12a, 12b, 12c et 12d sont des schémas illustrant la mesure d'apoptose par cytométrie en flux. La figure 12a illustre les cellules ayant subient aucun traitement. La figure 12b illustre des cellules misent en présence de 5FU à une concentration de 100 $\mu$g/ml. La figure 12c illustre des cellules misent en présence avec des bactéries lactiques à une concentration de $10^8$. La figure 12d illustre la combinaison de cellules, de bactéries lactiques ($10^8$) et de 5FU (100 $\mu$g/ml).

Figure 13 montre un western blot illustrant l'activation de la caspase 3 par des cellules misent en présence d'une composition selon un mode préféré de l'invention.

Figure 14 est un graphique montrant la mesure de l'apoptose des cellules LS 513 en présence de compositions selon un mode préféré de l'invention.

Figure 15 est un graphique montrant la mesure de la viabilité des cellules LS 513 par le MTT.

Figure 16 est un graphique montrant l'effet des bactéries lactiques vivantes versus les bactéries chauffées sur l'apoptose des cellules LS 513.

Figure 17 montre des western blots illustrant l'effet des bactéries lactiques vivantes versus les bactéries chauffées sur l'activation de la caspase 3.

Figure 18 est un graphique montrant la mesure d'apoptose inhérente aux souches bactériennes lactiques de la présente invention.

Figure 19 est un graphique montrant l'effet apoptotique du mélange de bactéries vivantes et de bactéries chauffées.

Figure 20 est un graphique montrant l'effet d'ajout d'acide butyrique et de 5FU sur l'apoptose des cellules LS 513.

Figure 21 est un graphique montrant l'effet de la composition selon un mode préféré de l'invention sur l'expression du récepteur Fas.

Figure 22 est un graphique montrant l'effet de la composition selon un mode préféré de l'invention sur l'expression du ligant Fas.

Figure 23 montre des western blots illustrant l'effet de la composition selon un mode préféré de l'invention sur l'expression d'une protéine impliquée dans l'apoptose, soit la protéine p53.

Figure 24 montrent des western blots illustrant l'effet de la composition selon un mode préféré de l'invention sur l'expression d'une protéine impliquée dans l'apoptose, soit la protéine p21

Figure 25 est un graphique montrant l'effet de PKC sur l'apoptose.

Figure 26 est un graphique montrant l'effet de l'inhibition de PKC sur l'apoptose.

La Figure 27 est un graphique illustrant l'effet des surnageants de bactéries lactiques de l'invention sur l'induction de l'activité de la caspase-3 dans les cellules LS 513.

La Figure 28 montre des photographies illustrant l'effet des surnageants des bactéries lactiques de l'invention sur la coloration en fluorescence du noyau des cellules LS 513.

La Figure 29 est un graphique illustrant l'effet des surnageants de bactéries lactiques de l'invention sur la viabilité des cellules LS 513.

La Figure 30 est un graphique illustrant l'effet des surnageants de bactéries lactiques de l'invention sur le gel cellulaire des cellules LS 513.

## DESCRIPTION DÉTAILLÉE DE L'INVENTION

La présente invention porte donc sur la mise en évidence de l'utilisation de propriétés nouvelles de la souche bactérienne lactique Lactobacillus acidophilus I-1492 déposée à la CNCM dans la prévention ou le traitement d'un cancer. Plus particulièrement, l'usage de ces bactéries lactiques vise à faciliter l'induction de l'apoptose cellulaires d'un cancer.

L'invention concerne aussi l'implication de cette souche bactérienne lactique dans des méthodes et compositions utiles dans le traitement ou la prévention d'un cancer, telle cancer du colon.

Selon un premier mode de réalisation, la présente invention vise l'utilisation de la souche bactérienne lactique Lactobacillus acidophilus I-1492 déposée à la CNCM pour renforcer la réponse immunitaire chez un mammifère dans le but de prévenir ou traiter un cancer.

Selon un second mode de réalisation, la présente invention vise l'utilisation de ladite bactérie lactique pour faciliter l'induction de l'apoptose des cellules d'un cancer. Par « faciliter l'induction de l'apoptose », on entend un procédé par lequel la présence des souches bactériennes lactiques de l'invention module positivement la mort cellulaire d'une tumeur, et préférablement une tumeur du colon.

Par « mammifère », on entend tout organisme vivant qui peut être sujet à un cancer, et ceci inclut les êtres vertébrés tels que notamment les êtres humains, les animaux domestiques et sauvages.

Par « traiter », on entend un procédé par lequel les symptômes du cancer, et particulièrement celui du colon, sont atténués ou complètement éliminés.

Par « prévenir », on entend un procédé par lequel le cancer, et particulièrement celui du colon, est enrayé ou retardé.

Selon un mode de réalisation préféré de l'invention, la souche bactérienne est sous une forme vivante ou irradiée.

Selon un troisième mode de réalisation, la présente invention porte sur l'utilisation de cette souche bactérienne lactique pour la préparation de compositions utile dans le traitement ou la prévention du cancer, tel le cancer du colon. Une composition selon la présente invention comprend une quantité efficace de ladite souche bactérienne lactique et un véhicule pharmaceutiquement acceptable. Préférablement, la composition de l'invention comprend un mélange de ladite souche de *L. acidophilus* et d'une souche de l'espèce *L. casel*.

Par "pharmaceutiquement acceptable", on entend un véhicule qui peut être administré sans risque à un mammifère, en particulier à un être humain, et ce avec peu ou sans effets secondaires négatifs ou toxiques. Un tel véhicule peut être utilisé pour différentes fonctions. Par exemple, il peut être utilisé en tant qu'agent de préservation, de solubilisation, stabilisant, émulsifiant, adoucissant, colorant, odorant, ou en tant qu'agent antioxydant. Ces types de véhicules peuvent être aisément préparés en utilisant des méthodes bien connues de l'homme de l'art.

Selon un mode de réalisation préféré, la composition de la présente invention comprend, en outre, un agent anticancéreux. À cet effet, tout agent anticancéreux pouvant être utiles dans le présent contexte est inclus dans la portée de la présente invention. Toutefois, le 5 fluoro-uracil est avantageusement utilisé en tant qu'agent anticancéreux. La composition de la présente invention peut également faire partie d'une formulation thérapeutique plus complexe, laquelle est utile dans le traitement et la prévention du cancer.

La quantité ou la concentration de bactéries lactiques qui est présente dans la composition de l'invention est une quantité thérapeutiquement efficace. Une quantité thérapeutiquement efficace de bactéries lactiques est cette quantité nécessaire pour obtenir des résultats positifs sans causer des effets secondaires excessivement négatifs chez l'hôte dans lequel les bactéries lactiques ou la composition est administrée. D'ailleurs, une quantité efficace de bactéries lactiques pour traiter un cancer particulier est une quantité qui est suffisante pour atténuer ou réduire d'une quelconque façon les symptômes liés au cancer. Une telle quantité peut être administrée en une seule dose ou peut être administrée selon un régime, par lequel elle est efficace. La quantité de bactéries lactiques selon la présente invention peut traiter le cancer mais, typiquement, elle est administrée afin d'atténuer les symptômes du cancer. La quantité exacte de bactéries lactiques ou de chacun des composants de la composition et la quantité de la composition à administrer varira selon des facteurs tels que le type de cancer à traiter, les autres ingrédients dans la composition, du mode d'administration, de l'âge et du poids du mammifère, etc...

Les compositions selon la présente invention peuvent se présenter sous une forme solide ou liquide quelconque habituelle pour l'administration pharmaceutique, c'est-à-dire par exemple des formes d'administration liquide, en gel, ou tout autre support connu de l'homme du métier. Parmi les compositions utilisables, on peut citer notamment les compositions administrables par voie orale. Dans ce cas précis, la composition de l'invention peut être administrée sous forme d'aliments ou de compléments alimentaires. On peut également cité des compositions injectables plus particulièrement destinées aux injections dans la circulation sanguine chez l'humain.

Une personne versée dans le domaine saura préparer des compositions pharmaceutiquement acceptables et déterminer, en fonction de plusieurs facteurs, le mode d'administration privilégié et la quantité devant être administrée. Parmi les facteurs pouvant influencer ses choix l'on retrouve: la nature du traitement, la nature exacte des ingrédients, actifs ou non, entrant dans la composition; le stade de la maladie; la condition, l'âge et le poids du patient, etc.

La présente invention inclut également les kits pharmaceutiques utiles, par exemple, pour la prévention ou le traitement d'un cancer, tel le cancer du colon. Les kits comportent un ou plusieurs récipients contenant, en outre, une composition selon la présente invention. De tels kits peuvent de plus inclure, si désirés, un ou plusieurs composants pharmaceutiques conventionnels, comme, par exemple, des récipients contenant un ou plusieurs véhicules pharmaceutiquement acceptables, ou toute autre composante additionnelle, qui sera évident à une personne de l'art. Un kit selon la présente invention peut avantageusement inclure des instructions sous forme de pamphlet ou sur tout autre support imprimé, indiquant les quantités des composantes à administrer, les directives pour l'administration, et/ou les directives pour mélanger les composantes.

L'exemple ci-après permettra de mettre en évidence d'autres caractéristiques et avantages de la présente invention.

## **EXEMPLE**

L'exemple qui suit sert à illustrer l'étendue de l'utilisation de la présente invention et non à limiter sa portée. Des modifications et variations peuvent y être effectuées sans que l'on échappe à l'esprit et à la portée de l'invention. Bien que l'on puisse utiliser d'autres méthodes ou produits équivalents à ceux que l'on retrouve ci-dessous pour tester ou réaliser la présente invention, le matériel et les méthodes préférés sont décrits.

### INTRODUCTION

Dans le contexte de la présente invention, afin de déterminer comment les bactéries lactiques aident à l'apoptose du cancer, des essais ont été faits sur la lignée cellulaire cancéreuse du colon humain LS-513. Les bactéries lactiques utilisées constituent un mélange de *Lactobacillus acidophilus* et *Lactobacillus casei*. L'agent anticancéreux est le 5 fluoro-uracil (5FU). Ce composé agit en tant qu'inhibiteur de l'enzyme synthétisant la thymine.

### MATÉRIEL ET MÉTHODES

### 1 Bactéries Lactiques

#### 1.1 Origine

Le mélange de bactéries utilisé pour les différentes expériences est fourni par la compagnie Bio-K (Laval, Qc, Canada). Le mélange comprend une combinaison de *Lactobacillus acidophilus* I-1492, qui fait l'objet de la demande international no. WO 98/23727; et de *Lactobacillus casei*.

#### **1.2 Préparation**

Les bactéries reçues dans 9 mL de milieu complexe MRS (Difco laboratories, Détroit, USA) sont aussitôt multipliées dans 100 mL du même milieu en prélevant 100 µL de la suspension bactérienne. Après une incubation de 18 heures dans un incubateur à 37°C, 10 mL de glycérol sont ajoutés au mélange de 100 mL qui est ensuite réparti en parties aliquotes de 1 mL dans plusieurs flacons de plastique stériles pouvant contenir 1,5 mL. Ces flacons sont entreposés dans un congélateur à la température de -80°C.

Pour les différents protocoles de stimulation, un flacon est dégelé et mis dans 9 mL de MRS et incubé pendant 18 heures à 37°C. Après ce temps d'incubation, un repiquage est effectué. Un volume de 100µL est prélevé et ajouté à 9 mL de MRS qui est également incubé pendant 18 heures à 37°C. Après ces incubations, les bactéries sont lavées deux fois dans du PBS stérile et récoltées par centrifugation à 3500 RPM pendant 10 minutes. Elles sont ensuite suspendues dans un volume final de 9 mL de RPMI stérile contenant 10% de sérum bovin foetal et sont ensuite prêtes à être utilisées pour une stimulation cellulaire. Lors des différentes expériences, les bactéries sont utilisées sous forme chauffées, irradiées et vivantes.

#### 1.3 Chauffage

Les bactéries sont chauffées pendant 40 secondes à "élevé" dans un four à micro-ondes (Général Électrique, Turntable Microwave Oven, 700 walt) afin d'obtenir une température de 100°C et produire le mélange de bactéries qui sera utilisé comme bactéries chauffées. Cette étape s'effectue dans un contenant de verre fermé.

#### 1.4 Irradiation

Afin de produire le mélange de bactéries irradiées, les tubes de bactéries vivantes sont irradiés à une dose minimale pour obtenir une létalité de 100%, soit de 5 kGy. Les mélanges sont irradiés dans un Gammacell-220 (MDS Nordion, Laval, Qc, Canada) utilisant le Cobalt-60 ($^{60}$Co) comme source émettrice de rayons gamma.

Afin de faire un comptage bactérien pour obtenir une concentration de bactéries par1 mL de cette suspension est ajouté à 9 mL d'eau peptonée, une solution isotonique contenant 0.1 % de bactopeptone (Difco laboratories, Detroit, USA). Des dilutions en séries sont effectuées. Ensuite, 1 mL de ces dilutions est prélevé et déposé dans un pétri auquel on ajoute 10 mL de MRS à 1.5% d'agar (Difco Laboratories, Detroit, USA) pour permettre d'effectuer un dénombrement après une incubation de 48 heures dans un incubateur à 37°C. Chaque échantillon est fait en duplicata.

<u>2 Cellules cancéreuses</u>

2.1 Origine

La lignée cellulaire LS 513 (ATCC, Rockville, MD, USA) est une lignée continue de cancer du colon d'origine humaine.

2.2 Culture

La lignée cellulaire étant adhérente, il est obligatoire de décoller les cellules, à l'aide d'une solution de trypsine-EDTA (Gibco, Burlingtion, ON, Canada) pour ensuite les resuspendre dans du RPMI supplémenté de L-glutamine et de 10% SBF, qu'on peut donc appeler "RPMI complet". Les plateaux sont faits la journée précédant la stimulation pour permettre aux cellules d'adhérer aux plateaux. Le jour de la stimulation, les différents produits sont ajoutés aux concentrations désirées et ensuite les cellules sont incubées pendant une période déterminée pour chaque type d'expérience, dans un incubateur à 37°C, 5% $CO_2$ et saturé en humidité.

<u>3 Co-cultures de cellules cancéreuses et de bactéries</u>

3.1 Ajout de bactéries

Une fois les plateaux cellulaires prêts à être utilisés, c'est-à-dire quand les cellules ont eu le temps d'adhérer, et les bactéries apprêtées pour être utilisées, celle-ci sont ajoutées selon le protocole de stimulation dans les puits contenant les cellules adhérées et le milieu "RPMI complet". Une incubation est effectuée pendant un temps déterminé en fonction de l'expérience à réaliser.

3.2 Expérience impliquant l'ajout de butyrate

Le plateau de cellules cancéreuses contenant 3 x 10$^5$ cellules par puits est incubé une nuit afin de permettre aux cellules d'adhérer. Ensuite, les différentes concentrations d'acide butyrique (Sigma, St-Louis, USA) sont ajoutées aux puits contenant les cellules adhérées et le milieu de culture, le RPMI complet. Suite à cet ajout, une incubation de 48 heures est faite afin de mesurer le pourcentage d'apoptose selon la technique décrite plus loin.

3.3 Récolte et études des surnageants

Le but de cette expérience est de vérifier si la présence de bactéries modifie la présentation pharmacologique du 5FU. Une première stimulation est effectuée sur des cellules adhérées pour une période de 48 heures en présence des différents produits de stimulation. Suite à cette incubation, les surnageants sont récoltés et ajoutés sur une nouvelle culture de cellules fraîchement adhérées. Ensuite, une seconde incubation de 48 heures est réalisée pour ensuite récolter les cellules et mesurer le pourcentage d'apoptose.

<u>4 Mesure de la viabilité des cellules cancéreuses</u>

4.1 Prolifération

4.1.1 MTT

Les cellules sont préparées comme indiqué plus haut. Le test s'effectue dans une plaque de 96 puits. Un volume de 100µL d'une concentration de 3,3 x 10$^5$ cellules par mL est déposé dans chaque puits, sauf dans la première rangée qui servira de "blanc". Une fois les produits de stimulation ajoutés, une incubation de 48 heures est effectuée. Suite à l'incubation, les surnageants sont retirés par aspiration, et le feuillet cellulaire est lavé, en ajoutant de façon délicate 200µL de PBS dans chaque puits, et en décantant de façon rapide le PBS ajouté dans l'évier. Ensuite, une solution de MTT (Sigma, St-Louis, USA) à 5X dilué dans du RPMI complet est ajouté dans les puits et une incubation de 5 heures à 37°C est effectuée. Par la suite, la solution est décantée et une autre solution est ajoutée dans le but de dissoudre les cristaux formés dans les cellules vivantes. Cette solution est composé de 50% de diméthyl formamide et 12% de sodium dodecyl sulfate (SDS). Une incubation de 18 heures est requise pour dissoudre tous les cristaux. Suite à cette incubation, la plaque est lue dans un spectrophotomètre (Mandel Scientific company, Bio-Tek instruments, Microplate EL309 Autoreader) à 540nM. Chaque échantillon est effectué en triplicatas, et une moyenne est faite à partir des valeurs obtenues. La valeur de la moyenne du témoin est le « 100% » de cellules vivantes. Pour obtenir le pourcentage des autres échantillons, il suffit d'effectuer un produit croisé.

4.2 Apoptose

4.2.1 Cytométrie en flux

Une concentration de $5x10^4$ cellules par mL, à raison de 6 mL par puits, est utilisée afin d'obtenir $3x10^5$ cellules par échantillon. Les cellules sont préparées comme indiqué plus haut. Les différents produits sont ajoutés dans les puits, et une incubation de 48 heures est faite.

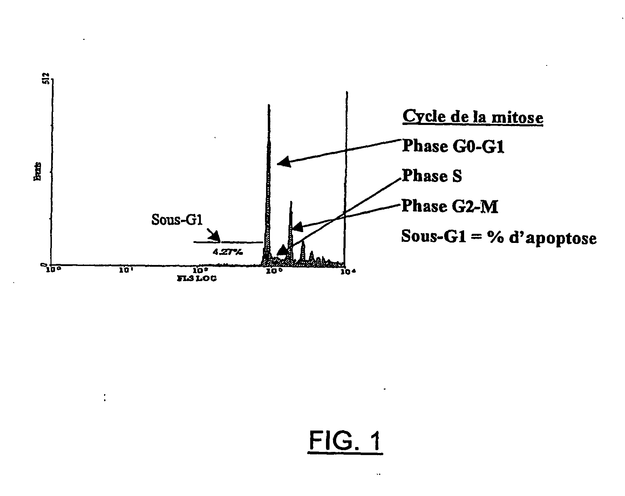

Suite à l'incubation, les surnageants des cellules sont récupérés dans différents tubes de 15 mL et centrifugés à 1500 RPM pendant 5 minutes. Les culots cellulaires sont ensuite récupérés par décantation des surnageants et mis de côté sur glace. Cette étape consiste à récupérer les cellules en suspension. Par la suite, le feuillet cellulaire adhéré est lavé avec 0,5 mL de trypsine-EDTA, puis 0,2 mL de trypsine-EDTA est ajouté dans chaque puits pour permettre aux cellules de se décoller lorsqu'elles sont incubées environ 8 minutes dans l'incubateur à 37°C. Les cellules sont suspendues dans 3 mL de RPMI complet 10% SVF pour arrêter l'activité enzymatique de la trypsine. La suspension cellulaire est centrifugée à 1500 RPM pendant 5 minutes dans les tubes servant pour la cytométrie en flux. Par la suite, les deux culots cellulaires (cellules en suspension et cellules adhérées) sont combinés et lavés deux fois avec du PBS froid supplémenté de 0,25% EDTA afin d'éviter la formation d'agglomérats de cellules. Suite aux lavages, 0,5 mL de solution d'iodure de propidium est ajouté au culot cellulaire. La solution est composée de 0,1% de citrate de sodium (Fisher Scientific, New Jersey, USA), 0,1% de TritonX-100 (Sigma, St-Louis, USA), 50μg/mL de RNase (Sigma, St-Louis, USA) et de 20μg/mL d'iodure de propidium (Sigma, St-Louis, USA). Une incubation de 15 minutes à 4°C est effectuée pour ensuite analyser les échantillons par cytométrie en flux (Coulter Epies XL-MCL). La Figure 1 résume un résultat typique d'une analyse par cytométrie en flux. Différents pics sont formés suite aux différences de fluorescence existant entre chaque étape du cycle cellulaire. Plus il y a un contenu élevé en ADN intact plus la fluorescence est élevée, et *vice versa*. Le programme mesure le pourcentage de fluorescence que forme le large pic situé sous le pic correspondant à G0-G1, ce pic représente des bouts d'ADN fragmentés, conséquence du clivage de l'ADN par différente enzyme activé lors de l'apoptose.

5 Mesure de l'expression de protéines impliquées dans l'apoptose (p53, p21, Caspase 3, Bax)

5.1 Buvardage de type Western

5.1.1 Stimulation et extraction des protéines

Un total d'environ $6x10^5$ cellules par échantillon est utilisé. Le lendemain, alors que les cellules sont devenues adhérentes, les produits de stimulation sont ajoutés. Les différents produits de stimulation sont le 5-Fluorouracile (100μg/mL) et les bactéries, vivantes ou chauffées, à une concentration de $1X10^8$ bactéries par mL. Ces produits vont amener les cellules à devenir apoptotique par la modulation de différentes protéines impliquées dans le processus. C'est cette modulation, augmentation de l'expression ou activation de protéine, que la technique permet de vérifier. Suite à différents temps de stimulation (puisqu'il s'agit d'une cinétique), les cellules sont récoltées et centrifugées à 1500 RPM pendant 5 minutes. Par la suite, 50μL de tampon de lyse composé de 50mM de Tris-HCl pH 7.5, NaCl 150 mM, Nonidet P-40 1% (Roche Diagnostics, Laval, Qc) ainsi qu'une pastille *Complète™* (contenant les inhibiteurs de protéases) (Roche Diagnostics, Laval, Qc), sont ajoutés au culot cellulaire qui est ensuite incubé 30 minutes sur glace. Le volume de 50 μL est prélevé et mis dans un micro-tube de 1,5 mL. Une centrifugation de 10 minutes à 15 000 RPM est effectuée afin de précipiter les débris cellulaires. Le surnageant est récupéré et un volume égal de "tampon d'échantillon" est ajouté. Le "tampon d'échantillon" est composé de 100mM Tris-HCl pH 6.8, 2% SDS, 20% glycérol et de 0,006% de bleu bromophénol. Pour terminer, les échantillons sont aliquotés par volume de 20μL et entreposés à une température de -20°C.

5.1.2 Séparation et identification des protéines

Les échantillons de protéines sont séparés sur un gel de polyacrylamide-SDS à 4% et 12% en utilisant la machine « mini-PROTEAN » de Bio-Rad. Les protéines migrent à l'intérieur d'un courant électrique de 200 volts pendant 45 minutes. La migration se fait dans un "tampon électrode" à pH 8,3, composé de 1,5 % Tris-Base, de 7,2% glycine et de 0,5 % SDS dans le l'eau mili-Q. Par la suite, les protéines sont transférées sur une membrane de nitrocellulose « Hybond ECL » (Amersham Pharmacia Biotech inc., Baie d'Urfé, Québec) à l'aide de la machine de transfert de Bio-Rad et ce pendant une heure à 100 volts dans un tampon de transfert composé de 0,58% Tris-Base, de 0,29% de glycine, de 0,037% SDS et de 20% méthanol. Suite au transfert, la membrane est "bloquée" avec une solution de blocage composée de PBS-Tween 80 à 0,1% ainsi que du lait en poudre écrémé à 5% pendant une heure à la température pièce et sous agitation. Ensuite, le premier marquage est effectué avec l'anticorps reconnaissant la protéine recherchée. L'anticorps est dilué dans la solution de blocage selon une concentration indiquée par le fournisseur. Après une heure de marquage, on effectue un lavage de 15 minutes suivi de deux lavages de 5 minutes, avec la solution de blocage. Le deuxième marquage est effectué avec un second anticorps qui reconnaît le premier anticorps et qui est couplé à la péroxidase. Une incubation d'une heure est faite avec ce second anticorps, qui est également dilué dans la solution de blocage à une concentration indiquée par le fournisseur. Dès que cette incubation est terminée, un lavage de 15 minutes, ainsi que deux lavages de 5 minutes, sont effectués avec une solution de PBS-Tween 80 à 0,1% sans lait en poudre écrémé. Afin de détecter les différentes protéines, une solution ECL (Amersham Pharmacia Biotech inc, Baie d'Urfé, Qc. Canada), qui amène l'activation de la peroxydase, est ajoutée sur la membrane selon les indications du fournisseur. Par la suite, les marquages sont révélés sur papier photographique (hyperfilm ECL, Amersham Pharmacia Biotech inc, Baie d'Urfé, Qc. Canada) qui sera marqué par la peroxydase activée. Le papier photographique est ensuite développé. Le tableau 1 résume les protéines recherchées et les réactifs utilisés.

Tableau 1 : Les anticorps utilisés pour les différentes protéines à identifier.

| Premier anticorps | | | |
|---|---|---|---|
| Protéine | Description | Dilution | Compagnie |
| p53 | Anti-p53 humain purifié Iso type :$IgG_{2a}$ souris | 1:1000 | BD PharMingen, Mississauga, Ontario |
| p21 | Anti-p21 souris purifié Iso type : $IgG_1$ souris | 2 :1000 | BD PharMingen, Mississauga, Ontario |
| Caspase 3 | Anti-capase 3 lapin polyclonal | 1 :1000 | BD PharMingen, Mississauga, Ontario |
| Bax | Anti-Bax | 1 :1000 | Santa Cruz California, USA |
| Deuxième anticorps | | | |
| IgG | Anti-IgG souris Couplé péroxidase Développé dans chèvre | 3 :10 000 | Sigma, St-Louis, USA |
| IgG | Anti-IgG lapin Couplé péroxidase Développé dans chèvre | 2.5 :10 000 | Sigma, St-Louis, USA |

6 Mesure de l'expression de margueurs sur la membrane cellulaire (Fas, Fas L)

6.1 Cytométrie en flux

Un total d'environ $5\times10^5$ cellules est utilisé par échantillon. Les cellules sont préparées comme mentionné précédemment. Une incubation de 24 heures est réalisée suite à l'ajout des différents produits de stimulation ( 5FU, bactéries vivantes ou chauffées et autres selon les expériences qui sont effectuées).

Suite à l'incubation, les feuillets cellulaires sont lavés avec 0,5 mL de trypsine-EDTA, puis 0,2 mL de trypsine-EDTA est ajouté aux feuillets de cellules, qui sont ensuite placés dans l'incubateur à 37°C pendant environ 10 minutes. Une fois les cellules décollées, 3 mL de RPMI complet sont ajoutés, et la suspension cellulaire est ensuite centrifugée pendant 5 minutes à 1500 RPM. Les surnageants sont décantés et les cellules sont placées sur.glace. Ensuite, $20\mu$L de la solution d'anticorps contre Fas récepteur (BD PharMingen, Mississauga, Ontario) ou Fas ligand (BD PharMingen, Mississauga, Ontario) sont ajoutés au culot de cellules, auquel on avait préalablement ajouté $50\mu L$ de "tampon pour cytométrie en flux" qui se compose de PBS 1X, 1% BSA, 0,02% d'azoture de sodium et 0,25% EDTA. Une incubation d'une demi-heure sur glace à la noirceur est requise. Ensuite, deux lavages avec 4 mL de "tampon pour cytométrie en flux" sont effectués, par centrifugation à 1500 RPM pendant 5 minutes. Aux cellules marquées par l'anticorps Fas ligand, une quantité de 0,25 $\mu L$ par tube de streptavidine-PE (BD PharMingen, Mississauga, Ontario) est ajoutée, suite à la première incubation et aux deux lavages. Une seconde incubation de 30 minutes sur glace à la noirceur est effectuée, suivie de deux autres lavages. À la fin d'un marquage, $250\mu L$ de solution de paraformaldéhyde (PBS 1x, 2% de para-formaldéhyde) et $250\mu L$ de "tampon de cytométrie en flux" sont ajoutés afin de fixer les marquages. Les tubes sont enveloppés dans du papier d'aluminium et placés à 4°C jusqu'à l'analyse des échantillons.

### 7 Mesure de l'expression du gène *Nurr77*

7.1 Extraction de l'ARN

Un nombre $3x10^5$ cellules est utilisé par échantillon. Une incubation de 3 heures est effectuée suite à l'ajout des différents produits de stimulation. Les cellules sont ensuite récoltées à l'aide d'un grattoir, puis centrifugées à 1500 RPM pendant 5 minutes. L'ARN total de chaque échantillon est extrait et purifié en utilisant la trousse High Pure RNA de Roche Diagnostics (Laval, Qc, Canada), tel qu'indiqué par le manufacturier. La concentration d'ARN est ensuite mesurée avec la machine (Pharmacia Biotech, Gene Quant RNA/DNA Calculator), puis ajustée à 92 $\eta g/\mu L$.

7.2 RT-PCR

La trousse LightCycler RNA amplification SYBR Green 1 de Roche Diagnostics (Laval, Qc, Canada) est utilisée afin de réaliser la réaction de transcription inverse (RT) et la réaction de polymérase en chaîne (PCR). Le principe du LightCycler est très semblable à celui du Thermocycleur. La différence majeure consiste en la possibilité, avec le LightCycler, d'observer l'amplification à chaque cycle, grâce à une molécule fluorescente appelée SYBR Green 1 qui s'insère dans chaque double brin formé. Plus il y a formation de doubles brins plus on observe, à l'aide du programme inclus avec le LightCycler, l'augmentation de la fluorescence. Les deux réactions, RT-PCR, se font dans un capillaire construit spécialement pour le LightCycler (Roche, Laval, Qc, Canada) et il suffit de faire un seul mélange des produits contenus dans la trousse et à utiliser cet ensemble selon les indications du manufacturier.

Avant d'effectuer une amplification, il est nécessaire de mettre au point certaines conditions. Par exemple, la concentration de $MgCl_2$, les températures, et le temps. Une concentration idéale de $MgCl_2$ est à déterminer. Pour l'amplification présente, une concentration de 7 mM s'est avérée la meilleure. La concentration des amorces est de 0,5 mM, comme le suggère le manufacturier. Dans le cas du gène *Nurr77*, la séquence des amorces utilisées pour le brin positif est 5'-CGACCCCCTGACCCCTGAGTT-3' et celle pour le brin négatif est 5'-GCCCTCAAGGTGTTGGAGAAGT-3' (Kang J-H, BioLPharm.Bull.). L'amplification par cette paire d'amorce donne 658 paires de bases. La programmation de la machine LightCycler est décrite dans le manuel d'instruction fourni par le manufacturier. Deux autres paramètres varient dans le programme d'amplification: entre autres dans le segment d'hybridation, la température à utiliser varie selon les amorces. La température (fixée à 5°C de moins que la température d'hybridation des amorces (Tm) se calcule par la formule suivante: Tm = 2°C (A+T) + 4°C (C+G). Pour les amorces utilisées la Tm est de 64°C. Le deuxième paramètre est le temps d'incubation pour l'élongation, toujours situé dans le programme amplification; il est déterminé par la formule suivante t = (nombre de paires de base du produit d'amplification $\div$ 25) secondes. Dans notre cas, le nombre de base étant 658, on obtient donc 26 secondes. Suite aux 35 cycles nécessaires pour amplifier la partie du gène voulu, une courbe de point de fusion est réalisée, utilisant une température de 10°C plus élevée que la température d'hybridation. Donc, les amplifications sont sujettes à une augmentation progressive de la température et à chaque degré la fluorescence est mesurée et enregistrée. En augmentant la température de façon progressive, les doubles brins formés se décollent lorsque la température devient assez élevée, et c'est donc à cette température qu'on observe une diminution de fluorescence. Le programme fait une courbe de point de fusion avec les fluorescences enregistrées, et par la suite, il mesure la dérivée de la fluorescence en fonction de la température. En connaissant la température de fusion théorique du produit d'amplification, il est possible d'obtenir la valeur de l'aire sous la courbe du pic formé par la séparation des brins du produit de l'amplification à cette température. La machine ne permettant pas de visualiser le nombre de paires de base amplifier, une migration sur gel d'agarose à 2% avec un marqueur de paire de base permet la vérification suite à la coloration au bromure d'éthidium à une concentration de 0,5 $\mu g/mL$ pendant 15 minutes.

### 8 Traitements par des inhibiteurs ou stimulateurs de la PKC

Les stimulations effectuées avec les inhibiteurs et stimulateurs de la PKC sont réalisées de la même façon que les stimulations par des bactéries et par le 5FU. Tout d'abord, les cellules sont préparées la veille pour leur permettre d'adhérer, et le jour même de la stimulation, les différents produits de stimulation sont ajoutés en même temps que l'inhibiteur GÖ 6976 (Sigma) et/ou tes stimulateurs de la PKC, ionomycine (Sigma) et PMA (phorbol 12-myristate 13-acetate) (Sigma). Ensuite, une incubation de 48 heures est effectuée, les cellules sont récoltées et le pourcentage d'apoptose est mesuré.

9 Dosage de cytokine

9.1 Dosage du TNF par bioessai

Il est possible de doser la quantité de TNF dans un surnageant à l'aide de la lignée cellulaire L929, qui est une lignée de fibroblastes de souris sensibles à l'action cytotoxique du TNF. Le principe de ce bioessai est simple : plus il y a de TNF dans le surnageant ajouté au feuillet de cellules L929, plus il y aura de mort cellulaire. On peut ensuite mesurer le taux de cellules restées vivantes. En premier lieu on cultive les cellules dans du RPMI 1640 complet + 5% SVF. Les cellules se décollent du flacon à l'aide de trypsine-EDTA (Gibco, Burlington, ON, Canada) en incubant environ 1 minute à 37°C. Un comptage cellulaire est effectué pour préparer une suspension de 3,3 x $10^5$ cellules/mL pour le bioessai.

Un volume de 75 $\mu$L est déposé dans chaque puits d'une plaque de 96 puits. Toutes les lignes reçoivent ce volume sauf la première qui est utilisée comme témoin vide. Suite à une incubation de 24 heures, un volume de 25 $\mu$L d'actinomycine D, à une concentration de 2 $\mu$g/mL, est ajouté à tous les puits de toutes les lignes, sauf la deuxième ligne qui sert de contrôle d'actinomycine D, et ce afin d'arrêter la croissance cellulaire.. Par la suite, les différents échantillons sont ajoutés à partir de la quatrième ligne à raison de 100 $\mu$L par puits, et ce en triplicata. La troisième ligne reste vide puisqu'elle servira de contrôle positif, c'est à dire qu'elle représentera le nombre maximum de cellules puisqu'il n'y a aucun agent cytotoxique ajouté. Avec les échantillons ajoutés, des dilutions successives sont effectuées à partir du 100 $\mu$L que l'on dilue en série dans les 8 rangées suivantes. Lorsque les échantillons sont dilués, on incube de 16 à 20 heures à 37°C + 5% $CO_2$. Après cette incubation, les surnageants sont rejetés et les cellules sont fixées au fond du puits à l'aide d'une solution de formaldéhyde 5% à raison de 100 $\mu$L par puits pendant 5 minutes. Les plaques sont vidées et rincées 3 fois à l'eau courante. Les cellules restées fixées sont ensuite colorées au crystal violet à raison de 50 $\mu$L par puits pendant 5 minutes. Par la suite, les plaques sont vidées et l'excès de colorant est éliminé par 3 rinçages à l'eau courante. Une fois les plaques bien sèches, 100 $\mu$L d'une solution d'acide acétique à 33% est ajoutée à chaque puits pour dissoudre de crystal violet absorbé par les cellules fixées. L'absorbance est lue à une longueur d'onde de 540 nm dans un spectrophotomètre pour plaque, en utilisant la colonne 1 comme référence (« blanc »).

Afin de calculer le nombre d'unités de TNF, on considère qu'une unité de TNF correspond à l'inverse du facteur de dilution donnant 50% de cytotoxicité. Afin de calculer le pourcentage de cytotoxicité pour chaque échantillon, l'équation suivante est utilisée.

$$\% \text{ cytotoxicité} = \frac{\text{D.O. échantillon}}{\text{D.O. positif}} \times 100$$

Donc, la D.O. de l'échantillon est la moyenne des trois absorbances obtenues pour une dilution suite à la lecture des plaques. La D.O. du contrôle positif est la moyenne des puits de la ligne 3. Le % de cytotoxicité est calculé pour chaque dilution. Par la suite, une droite à régression linéaire est tracée pour les dilutions d'un échantillon, le facteur de dilution (= x) et le % de cytotoxicité (= y) et le point 50% est trouvé à l'aide de l'équation de la droite. L'inverse de la dilution ($2^x$) équivaut au nombre d'unité de TNF dans l'échantillon initial non dilué. Les résultats sont exprimés en U/mL.

RÉSULTATS

1) Recherche de la dose de 5 Fluoro-uracil optimale

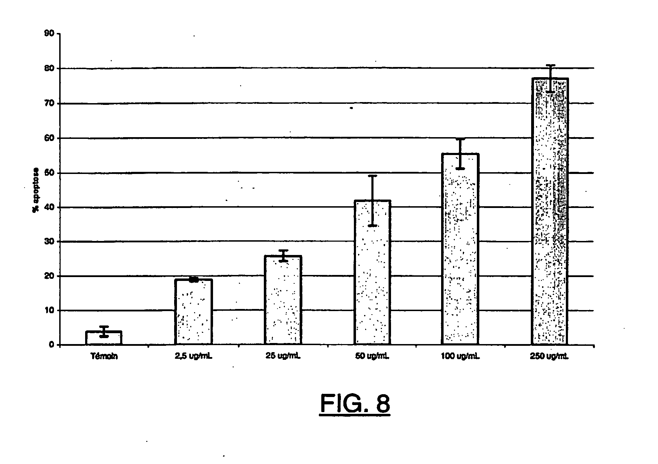

La Figure 8 montre la mesure de l'apoptose par cytométrie en flux suite à l'exposition de doses croissantes de 5-Fluoro-uracil (5FU) afin d'obtenir une concentration idéale donnant 50% de mortalité. Un total de $3 \times 10^5$ cellules est mis en présence des différentes concentrations de 5FU pendant 48 heures. Ensuite, le contenue d'ADN des cellules est marqué à l'aide d'iodure de propidium et analysé par cytométrie en flux afin d'obtenir le pourcentage d'apoptose. Les résultats suivants sont la moyenne de deux expériences indépendantes.

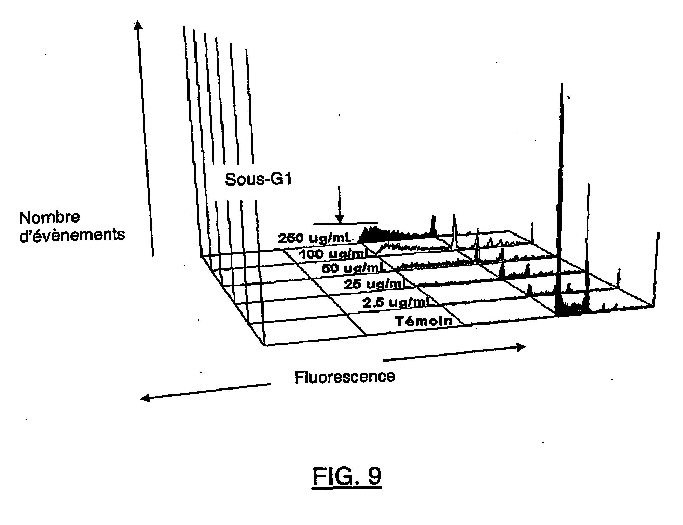

La Figure 9 illustre un schéma représentant une des deux expériences effectuées afin d'obtenir la concentration idéale de 5FU. L'appareil mesure le nombre d'événement du pic sous-G1 par rapport au nombre d'événement de l'échantillon, ce qui donne un résultat en pourcentage. Puisque l'on sait que le pic sous-G1 correspond à de l'ADN clivée, signe d'apoptose, on donne donc à se pourcentage la valeur de l'apoptose.

2) Action de la combinaison de 5 Fluoro-Uracil + bactéries vivantes sur la viabilité des cellules LS 513.

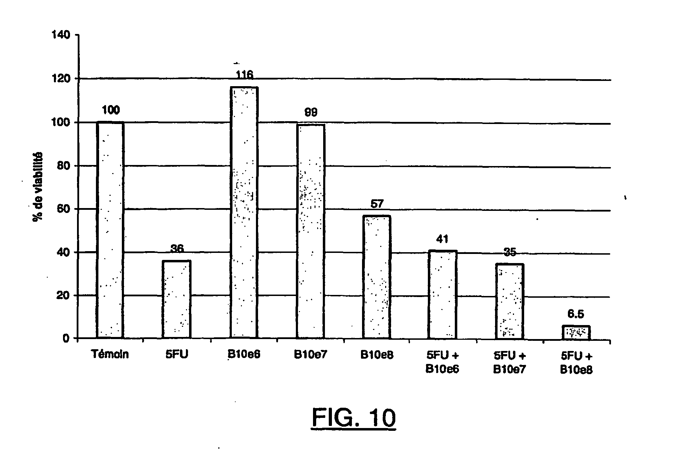

La Figure 10 illustre la viabilité des cellules cancéreuses du colon est dosée par le MTT suite à une incubation de 48 heures en présence ou absence de 5-Fluoro-uracil (5FU) ( 2.5ug/mL) et de bactéries vivantes (B) à différentes concentrations ($10^6$-$10^8$). Un total de 3.3 X $10^4$ cellules par puits dans un plateau de 96 puits est mis en contact avec les différents produits de stimulation. La coloration produite par la réaction du MTT avec les cellules vivantes, est évaluée sur un spectrophotomètre à une longueur d'onde de 540 nm. Chaque échantillon est la moyenne de 3 puits différents.

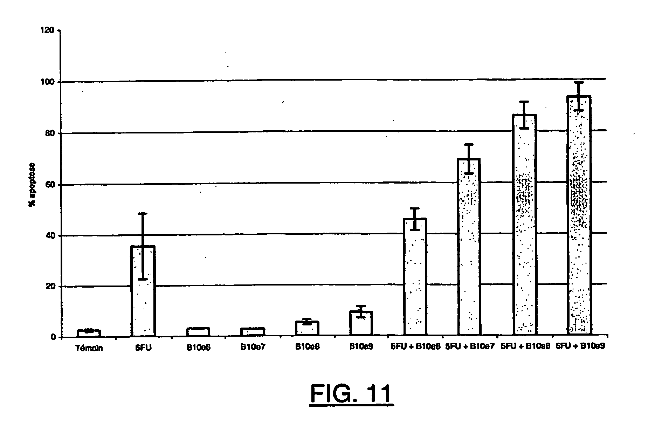

La Figure 11 illustre l'effet des bactéries vivantes et du 5FU sur l'apoptose de LS 513. Des bactéries lactiques vivantes (B) à différentes concentrations ($10^6$-$10^9$) et du 5-Fluoro-uracil (5FU) (100ug/mL) sont ajoutés aux cellules LS 513. La mesure de l'apoptose par cytométrie en flux est faite suite à une incubation de 48 heures. Le marquage de l'ADN avec une solution d'iodure de propidium permet d'observer le pourcentage de cellules ayant de l'ADN clivée (sous-G1) produite suite à l'incubation. Le témoin sont des cellules ayant subit aucun traitement.

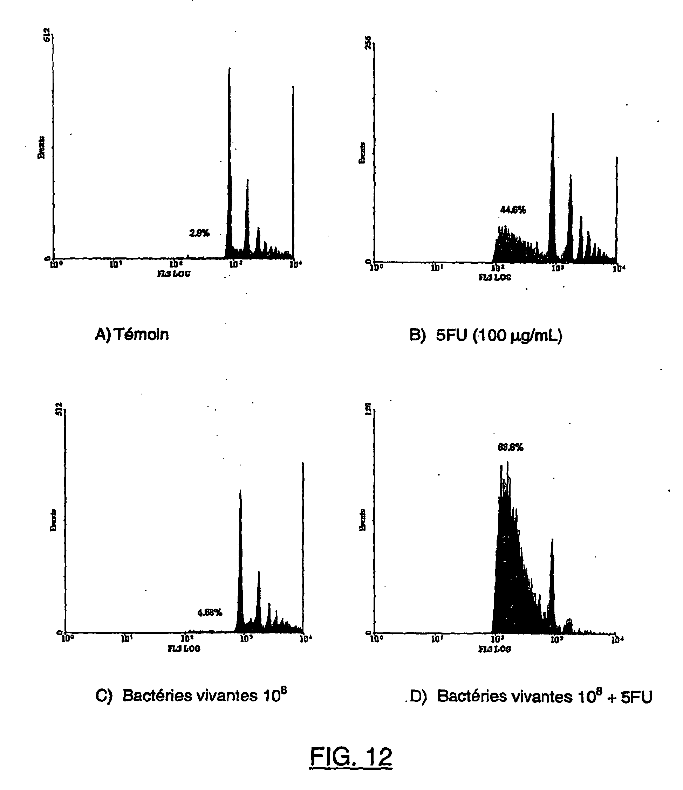

La figure 12 montre des exemples de schémas par cytométries en flux pour la mesure d'apoptose. Ces 4 schémas représentent 4 échantillons différents produit lors d'une expérience. Le nombre d'évènements en sous-G1 donne un pourcentage par rapport au reste des étapes du cycle de mitose. Le Témoin (A) étant les cellules ayant subis aucun traitement, l'image B est composé de cellules misent en présence de 5FU à une concentration de 100 $\mu$g/mL, l'image C est celui où on a combiné les bactéries vivantes, à une concentration de $10^8$, aux cellules et la dernière image (D), représente la combinaison de cellules, de bactéries vivantes ($10^8$) et de 5FU (100 $\mu$g/mL).

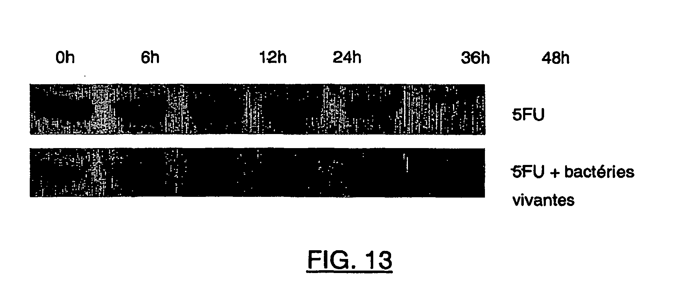

La Figure 13 illustre un western blot de la caspase 3 et ce sous sa forme pro-active. Une incubation de 48h en présence de bactéries vivantes à une concentration de $10^8$ et de 5-Fluoro-Uracil (5FU) à une concentration de 100ng/mL fut effectuée. De cette incubation, les protéines furent extraites et migrée sur gel de polyacrylamide. Par la suite, elles furent transférées sur membrane de nitrocellulose et marquées avec un anticorps spécifique pour la caspase 3 et ce à une concentration de 1:1000.

3) Action de l'état des bactéries

3.1) Bactéries vivantes versus bactéries irradiées

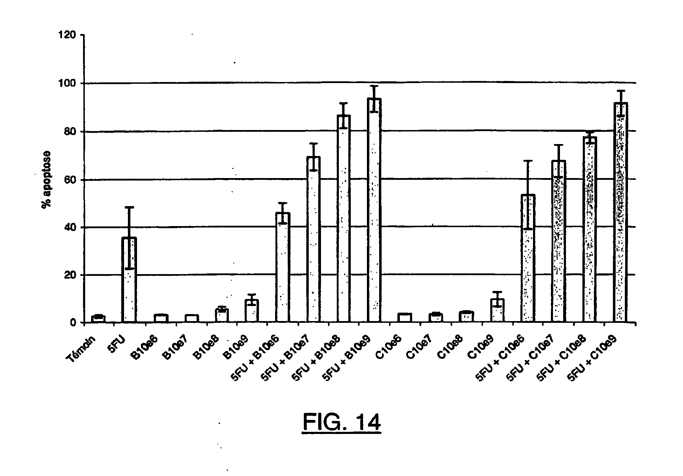

La Figure 14 montre la mesure de l'apoptose des cellules LS 513 en présence de bactéries vivantes, bactéries irradiées et de 5FU. Cette Figure montre plus spécifiquement la mesure de l'apoptose par le pourcentage de sous-G1 par cytométrie en flux à l'aide d'un marquage d'iodure de propidium (20 ug/mL). Les cellules du cancer du colon sont misent en présence ou absence de 5-Fluoro-Uracil (5FU) (100 ug/mL) et de bactéries vivantes (B) ou de bactéries irradiées (C) à différentes concentrations ($10^6$-$10^9$) et ce pour une période de 48 heures. Les témoins sont des cellules sans traitement.

3.2) Bactéries vivantes versus bactéries chauffées

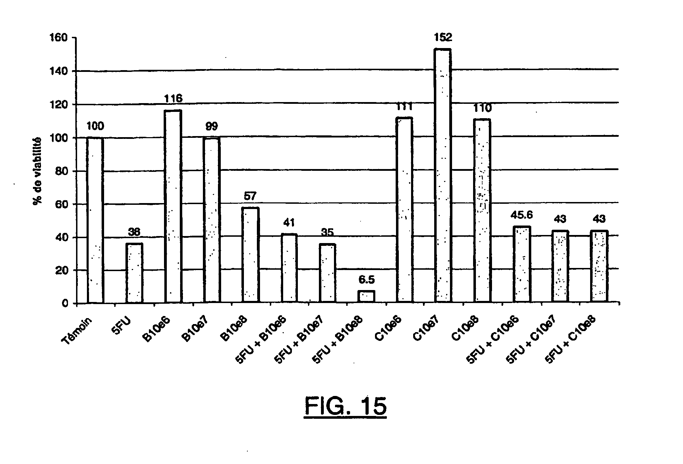

La Figure 15 montre la mesure de la viabilité des cellules LS 513 par le MTT. Plus particulièrement; cette Figure montre l'effet de bactéries vivantes (B) et de bactéries chauffées (C) à différentes concentrations (10eX) en présence ou absence de 5-Fluoro-Uracil (5FU) (A)(2.5ug/mL) sur la viabilité de LS 513 après une incubation de 48 heures. Les valeurs sont obtenues par lecture au spectrophotomètre (540nm), par la coloration due au MTT qui colore les mitochondries fonctionnelles donc celles des cellules vivantes seulement. La concentration cellulaire utilisée est de 3.3 x$10^4$ cellules par puit. Les résultats suivants sont la moyenne de trois puits d'un plateau de 96 puits.

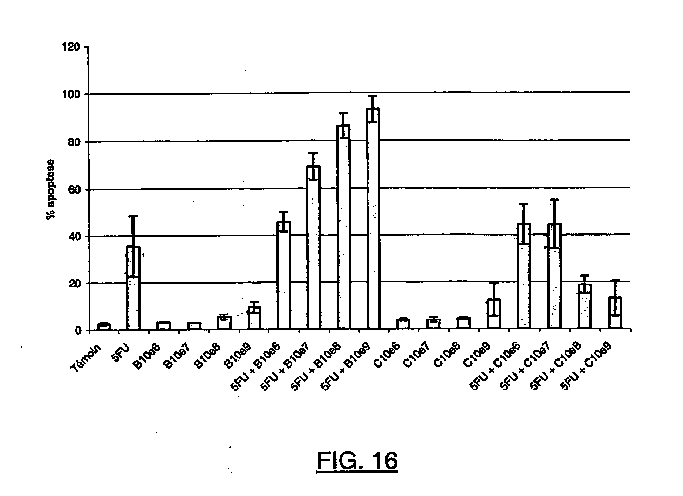

La Figure 16 montre l'effet des bactéries vivantes versus les bactéries chauffées sur l'apoptose des cellules LS 513. L'analyse du pourcentage de sous-G1 par marquage de l'ADN a été obtenu avec de l'iodure de propidium. Un total 10 000 événements est traité par échantillon. Une incubation de 48 heures en présence de bactéries vivantes (B) et de bactéries chauffées (C) et ce à différentes concentrations avec ou sans 5 Fluoro-Uracil (100ug/mL) sont les différents échantillons illustrés dans la figure.

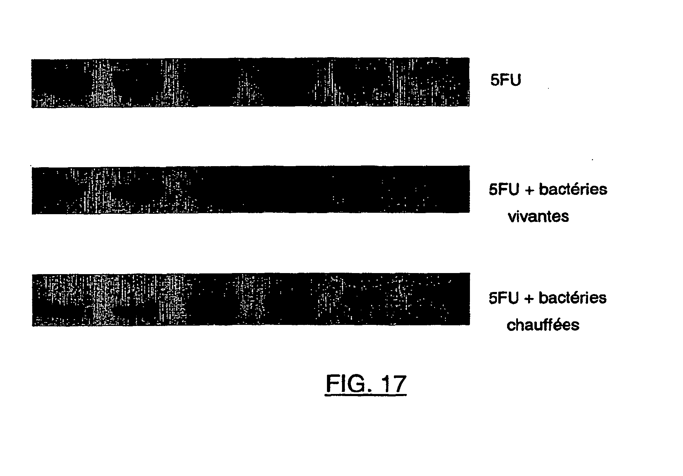

La Figure 17 montre l'effet des bactéries vivantes ou chauffées sur l'activation de la caspase 3.

4) Mécanismes possibles inhérents aux cultures bactériennes

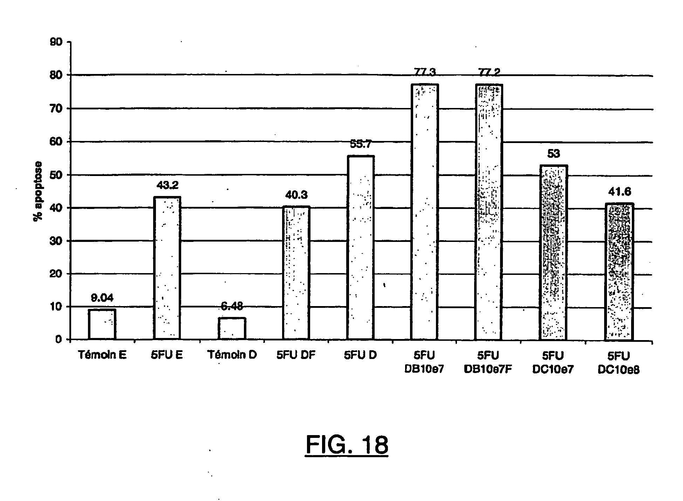

La Figure 18 montre la mesure d'apoptose inhérente aux souches de bactéries lactiques de l'invention. Une première stimulation fut effectuée pendant 48 heures. Certain puits ne contenaient pas de cellules (F). Les différents surnageas (D) ont été récoltés et ajoutés sur une culture cellulaire fraîche (E). La concentration de 5-Fluoro-uracil (5FU) est de 100 ug/mL et deux concentrations différentes sont utilisées pour les bactéries vivantes (B) et les bactéries chauffées (C) lesquelles sont $1 \times 10^7$ et $10^8$. La mesure de l'apoptose est faite par cytométrie en flux par marquage de l'ADN avec de l'iodure de propidium.

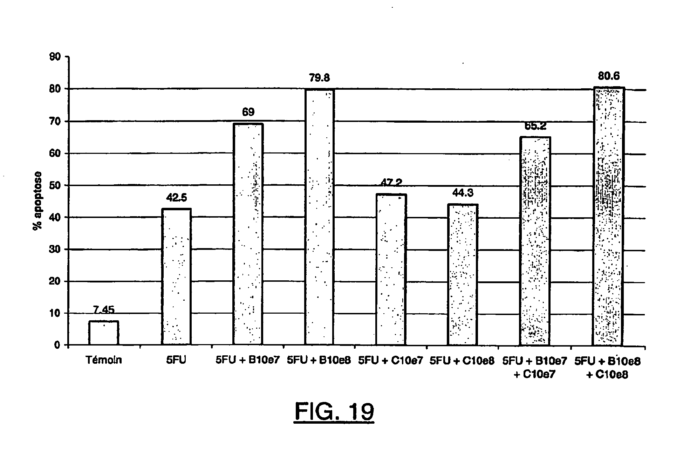

La Figure 19 montre l'effet apoptotique du mélange de bactéries vivantes et de bactéries chauffées. La mesure de l'apoptose par cytométrie en flux a été prise suite à une incubation de 48 heures. La lignée cellulaire LS 513 est mise en présence d'une concentration déterminée de 5FU (100ug/mL) ainsi que la présence de bactéries vivantes (B) et de bactéries chauffées (C) à deux concentrations différentes ($10^7$ et $10^8$). Le témoin est composé de cellules sans aucun traitement.

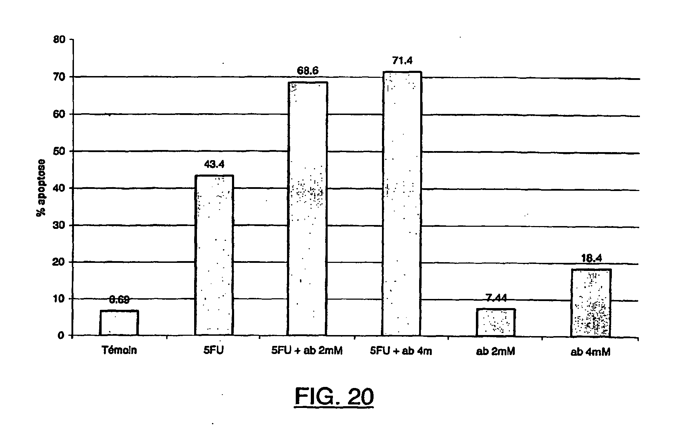

La Figure 20 montre l'effet de l'ajout d'acide butyrique et de 5FU sur l'apoptose des cellules LS 513. Aux cellules cancéreuses du colon est ajouté une dose de 5FU (100ug/mL) ainsi que différentes doses (2mM et 4mM) d'acide butyrique (ab). L'apoptose est mesurée suite à une incubation de 48 heures.

5) Mécanisme possible inhérent aux cellules tumorales

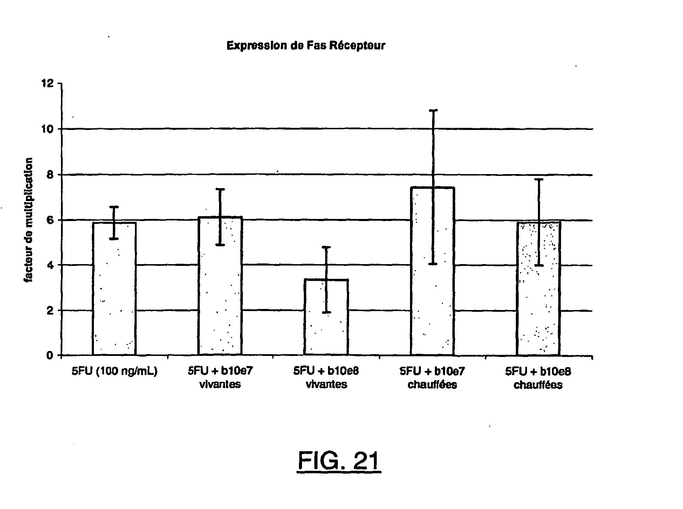

La Figure 21 montre l'effet de la composition selon un mode de réalisation préféré sur l'expression du récepteur Fas.

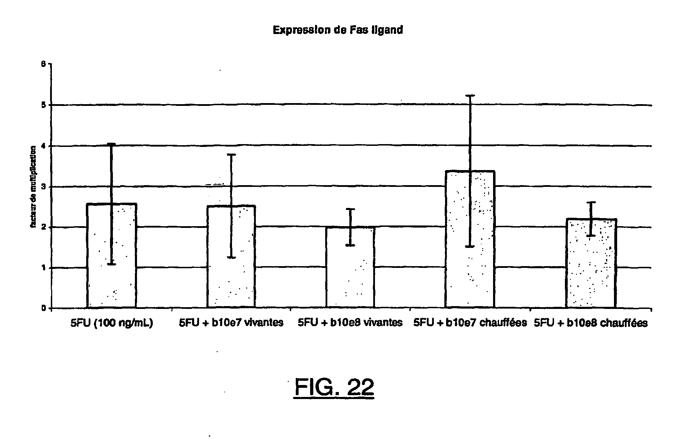

La Figure 22 montre l'effet de l'expression du Fas ligand.

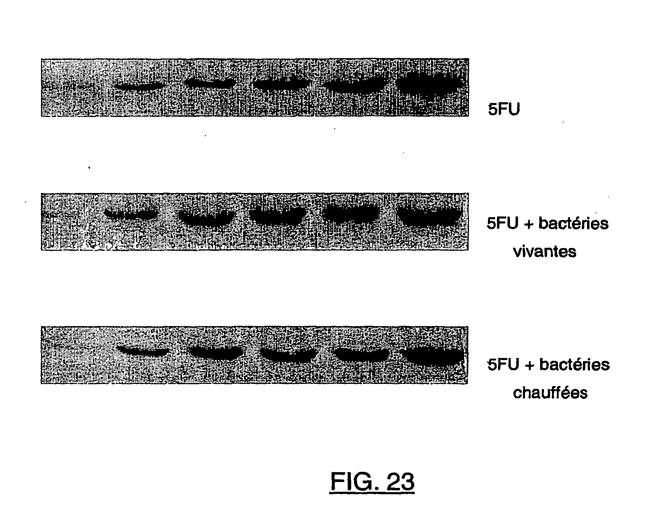

La Figure 23 illustre l'effet de la composition selon un mode préféré de l'invention sur l'expression de la protéine p53.

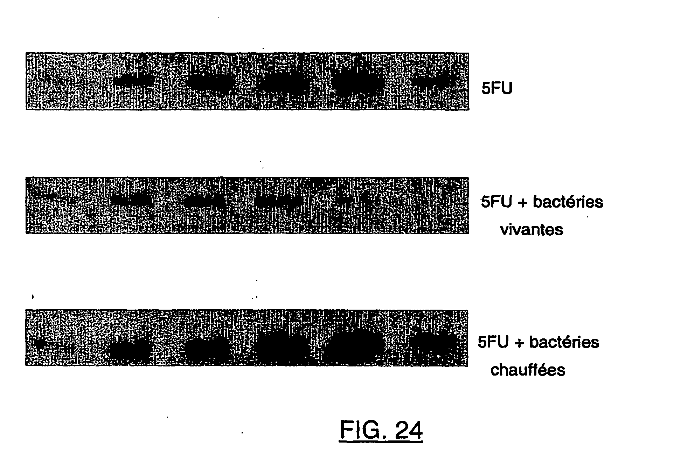

La Figure 24 illustre l'effet de la composition selon un mode préféré de l'invention sur l'expression de la protéine p21.

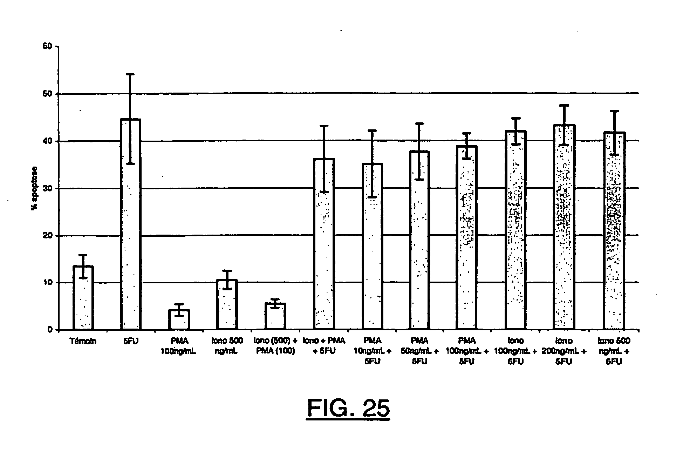

La Figure 25 illustre l'effet de l'activation de PKC sur l'apoptose.

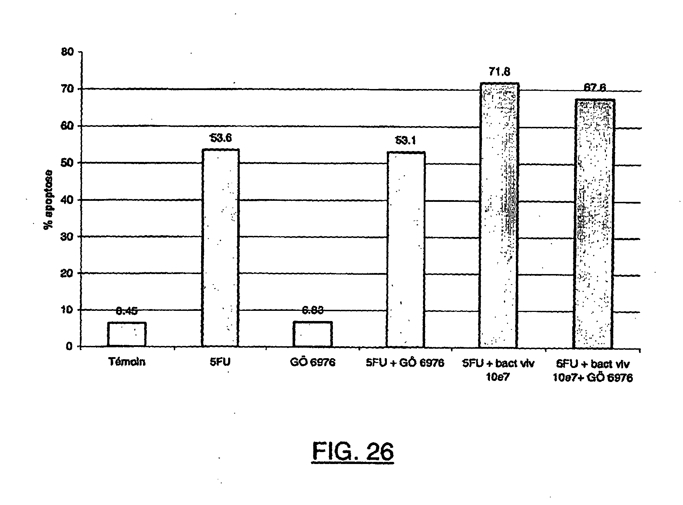

La Figure 26 illustre l'effet de l'inhibition de PKC sur l'apoptose.

6) Caractérisation du surnageant des bactéries lactiques de l'invention sur l'apoptose des cellules de tumeurs intestinales LS 513

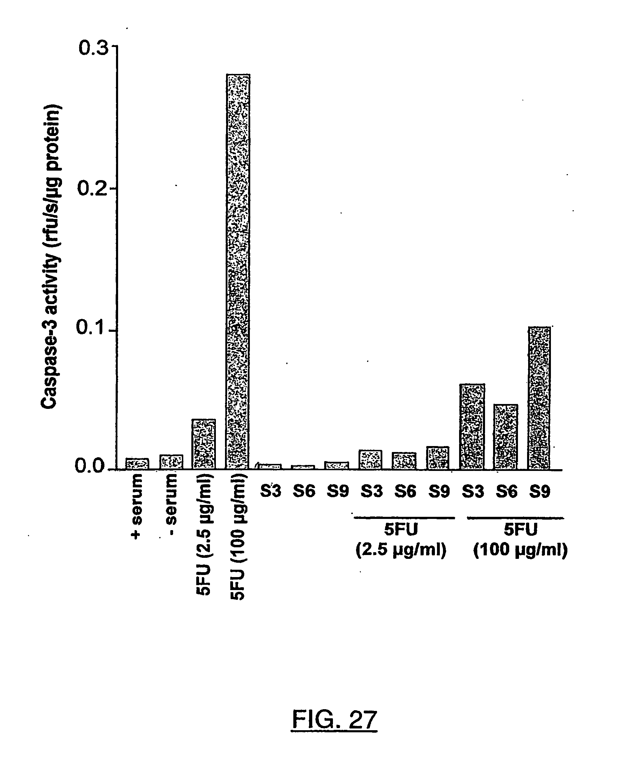

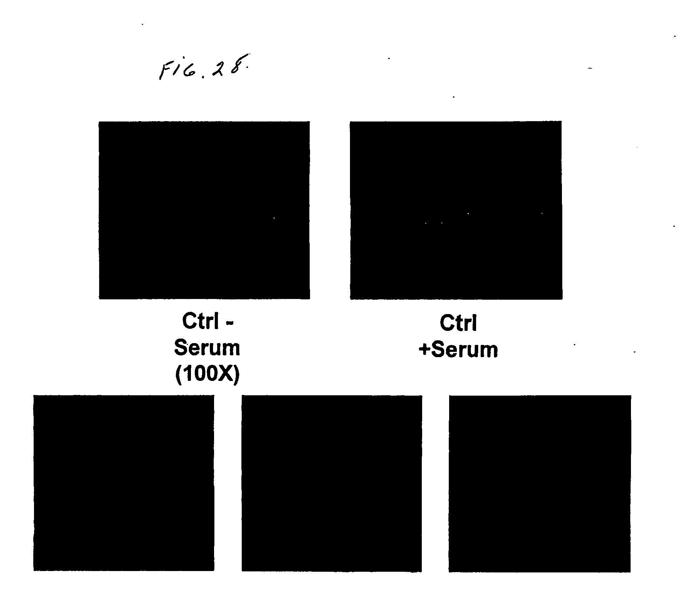

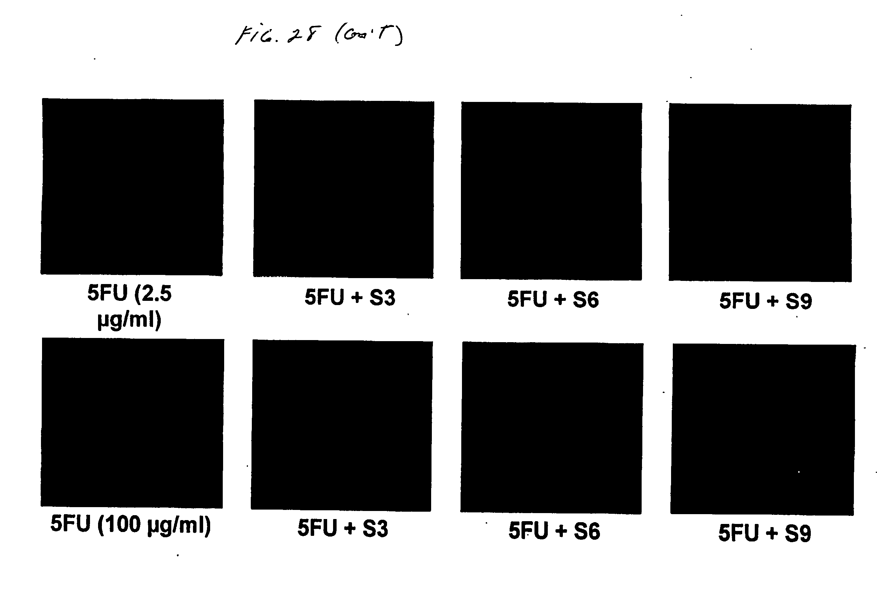

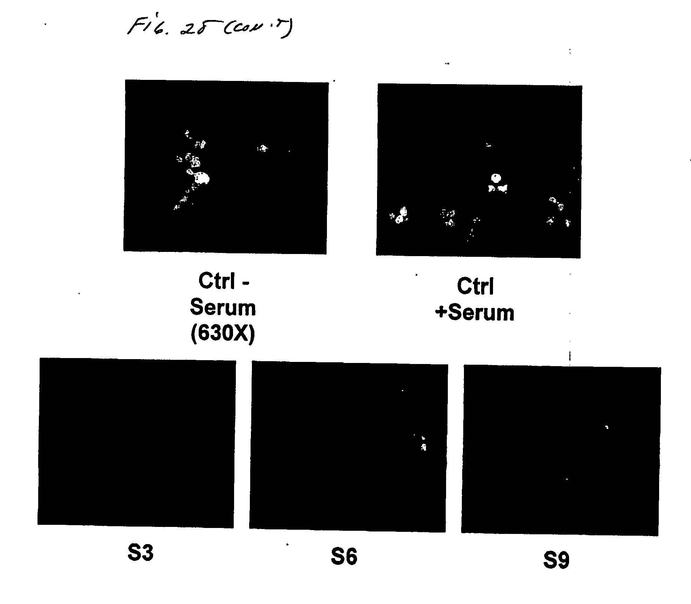

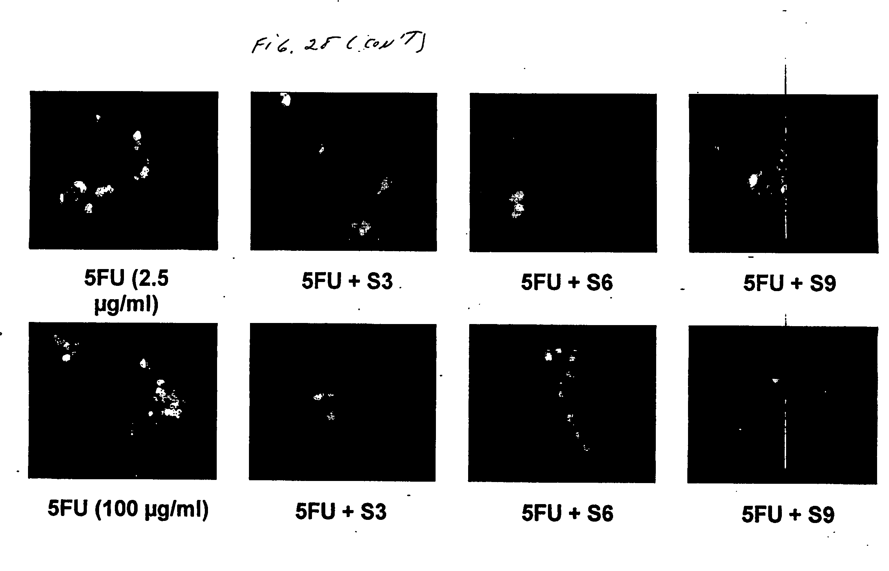

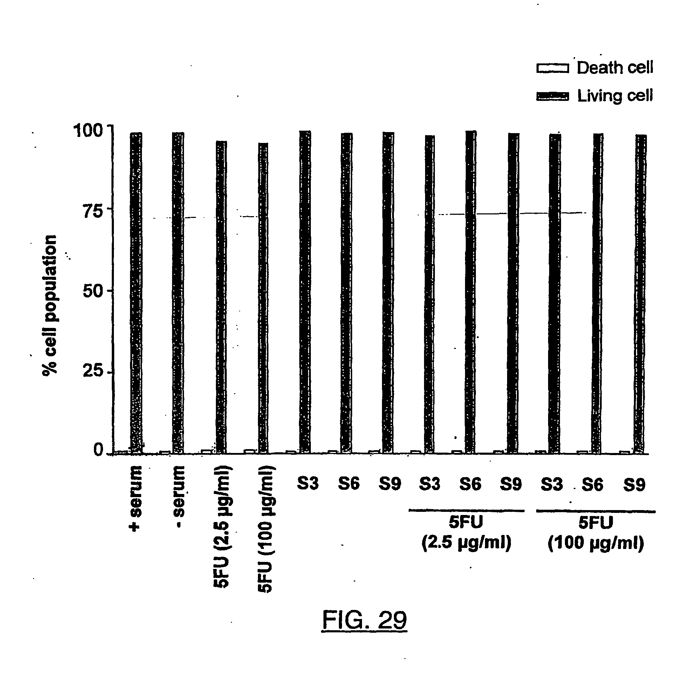

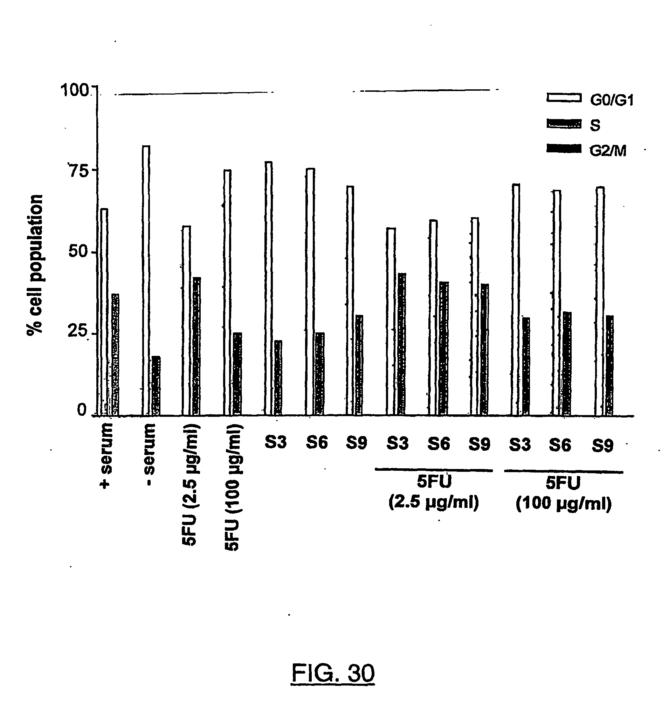

Le potentiel apoptotique des surnageants de bactéries sur la lignée LS 513, lignée tumorale a été analysé. Les résultats obtenus sur l'activité de la caspase-3 démontrent que les surnageants, même en présence de 5-FluoroUracil ($2,5 \ \mu g/ml$ et $100 \ \mu g/ml$), n'activent pas de façon significative cette caspase (Figure 27). Au contraire, en présence de $100 \ \mu g/ml$ de 5FU, les surnageants inhibent l'activité de la caspase-3. La coloration en fluorescence du noyau des cellules cancéreuses, à l'aide de la technique du DAPI, n'a pas permis de mettre en évidence des noyaux où la chromatine est condensée, une caractéristique fondamentale des cellules apopotitiques. La coloration des noyaux est typique de cellules saines et vivantes (Figure 28, grossissement à 100X et à 630X). De plus, les études en cytométrie de flux à l'aide d'iodure de propidium ne démontrent pas de mort cellulaire (Figure 29) ni de perturbation significative des phases du cycle cellulaire (Figure 30). L'ensemble de ces résultats indiquent que les surnageants de bactéries n'induisent pas l'apoptose dans les cellules LS 513.

DISCUSSION

L'action des bactéries intactes c'est-à-dire celles qui sont vivantes et irradiées sur le cancer est la même. Par contre les bactéries non intactes, par exemple celles détruites par la chaleur semblent avoir une action contraire aux bactéries intactes.

De plus, il a été remarqué selon les travaux effectués dans le contexte de la présente invention que l'efficacité d'un agent anticancéreux tel que le 5 FU est considérablement augmentée en présence de bactéries intactes. Cette efficacité serait dose dépendante. Une apoptose plus rapide en présence de bactéries vivantes est remarqué.

La présence de bactéries chauffées avec le 5 FU augmenterait l'expression de ia protéine p21. Il pourrait donc y avoir une modulation via un récepteur inconnu de protéines régulatrices de l'apoptose\cycle cellulaire (p21). Une augmentation de la protéine p21 a été remarquée lorsqu'il y a moins d'apoptose, et une diminution de la protéine lorsqu'il y a plus d'apoptose.

L'acide butyrique est un produit des bactéries lactiques et est pressente dans l'intestin. Ce produit cause l'apoptose dans les cellules du cancer du colon in vitro. Il y aura une synergie entre le butyrate et le 5FU sur le cancer du colon. Il a également été remarque que l'acide butyrique inhibe la croissance in vivo de cancer du colon humain sur les souris.

Au vu de ce qui précèdent, les bactéries lactiques vivantes entrent en synergie avec le 5FU pour diminuer le nombre de cellules cancéreuses en culture (MTT) ou bien pour augmenter l'apoptose chez ces dernières (cytosine en flux).

Les bactéries lactiques irradiées ont la même action que les bactéries vivantes alors que les bactéries chauffées auraient une action contraire. Ainsi, la forme intacte des bactéries lactiques est nécessaire à leur action contre les cellules tumorales. De plus, l'action des bactéries est également dépendante et proportionnelle à la dose. La propriété d'induire ou moduler l'apoptose par les bactéries lactiques de l'invention a été corroborée par l'expérience démontrant l'effet non-apoptotique du surnageant de ces bactéries.

L'expression de la caspase 3 ainsi que celle de la protéine p21 est modulée par les bactéries lactiques vivantes de la même façon que l'apoptose.

**Revendications**

1. Utilisation d'une souche bactérienne lactique pour la fabrication d'un médicament pour renforcer une réponse immunitaire chez un mammifère dans le but de prévenir ou traiter un cancer, ladite souche étant *Lactobacillus acidophilus* I-1492 déposée à la CNCM.

2. Utilisation selon la revendication 1, **caractérisée en ce que** ledit mammifère est un être humain.

3. Utilisation d'une souche bactérienne lactique pour la fabrication d'un médicament pour faciliter l'induction de l'apoptose des cellules d'un cancer, ladite souche étant *Lactobacillus acidophilus* I-1492 déposée à la CNCM.

4. Utilisation d'une souche bactérienne lactique pour la fabrication d'un médicament destiné au traitement ou la prévention du cancer, ladite souche étant *Lactobacillus acidophilus* I-1492 déposée à la CNCM.

5. Utilisation selon l'une quelconque des revendications 1 à 4, **caractérisée en ce que** la souche bactérienne est sous une forme vivante ou non-vivante mais intacte.

6. Utilisation selon l'une quelconque des revendications 1 à 5, **caractérisée en ce que** l'on utilise en outre une souche de l'espèce *Lactobacillus casei*.

7. Utilisation selon l'une quelconque des revendications 1 à 6, avec un agent anticancéreux.

8. Utilisation selon la revendication 7, **caractérisée en ce que** l'agent anticancéreux est le le 5 fluoro-uracil.

9. Utilisation selon l'une quelconque des revendications 1 à 8, **caractérisée en ce que** ledit cancer est un cancer du colon.

10. Composition pour traiter ou prévenir un cancer, comprenant une quantité efficace d'une souche bactérienne lactique et un véhicule pharmaceutiquement acceptable, ladite souche étant *Lactobacillus acidophilus* I-1492 déposée à la CNCM.

11. Composition selon la revendication 10, **caractérisée en ce que** la souche bactérienne est sous une forme vivante ou non-vivante mais intacte.

12. Composition selon l'une des revendications 10 ou 11, **caractérisée en ce qu'**elle comprend en outre une souche de l'espèce *Lactobacillus casei*.

13. Composition selon l'un quelconque des revendications 10 à 12, **caractérisée en ce qu'**elle comprend un agent anticancéreux.

14. Composition selon la revendication 13, **caractérisée en ce que** l'agent anticancéreux est le 5 fluoro-uracil.

15. Composition selon l'une quelconque des revendications 10 à 14, **caractérisée en ce que** ledit cancer est un cancer du colon.

16. Kit pour prévenir ou traiter un cancer chez un mammifère, **caractérisée en ce qu'**il comprend un récipient contenant une composition tel que définie aux revendications 10 à 14.

**Claims**

1. Use of a lactic acid bacterial strain for the manufacture of a medicament for strengthening an immune response in a mammal for the purpose of preventing or treating a cancer, said strain being *Lactobacillus acidophilus* I-1492 deposited in the CNCM.

2. Use according to Claim 1, **characterized in that** said mammal is a human being.

3. Use of a lactic acid bacterial strain for the manufacture of a medicament for facilitating the induction of apoptosis in the cells of a cancer, said strain being *Lactobacillus acidophilus* 1-1492 deposited in the CNCM.

4. Use of a lactic acid bacterial strain for the manufacture of a medicament for the treatment or prevention of cancer, said strain being *Lactobacillus acidophilus* I-1492 deposited in the CNCM.

5. Use according to any one of Claims 1 to 4, **characterized in that** the bacterial strain is in a living or non-living but intact form.

6. Use according to any one of Claims 1 to 5, **characterized in that** a strain of the species *Lactobacillus casei* is also used.

7. Use according to any one of Claims 1 to 6 with an anticancer agent.

8. Use according to Claim 7, **characterized in that** the anticancer agent is 5-fluorouracil.

9. Use according to any one of Claims 1 to 8, **characterized in that** said cancer is a colon cancer.

10. Composition for treating or preventing a cancer, comprising an effective amount of a lactic acid bacterial strain and a pharmaceutically acceptable vehicle, said strain being *Lactobacillus acidophilus* 1-1492 deposited in the CNCM.

11. Composition according to Claim 10, **characterized in that** the bacterial strain is in a living or non-living but intact form.

12. Composition according to Claim 10 or 11, **characterized in that** it also comprises a strain of the species *Lactobacillus casei.*

13. Composition according to any one of Claims 10 to 12, **characterized in that** it comprises an anticancer agent.

14. Composition according to Claim 13, **characterized in that** the anticancer agent is 5-fluorouracil.

15. Composition according to any one of Claims 10 to 14, **characterized in that** said cancer is a colon cancer.

16. Kit for preventing or treating a cancer in a mammal, **characterized in that** it comprises a receptacle containing a composition as defined in Claims 10 to 14.

**Patentansprüche**

1. Verwendung eines Milchsäurebakterienstammes zur Herstellung eines Medikamentes zur Verstärkung der Immunantwort bei Mammaliern zur Vorbeugung oder Behandlung von Krebs, wobei der Stamm *Lactobacillus acidophilus* I-1492 darstellt, der bei der CNCM (Collection Nationale de Cultures Micro-organismes, Institut Pasteur, Paris, France) hinterlegt ist.

2. Die Verwendung gemäß Anspruch 1, **dadurch gekennzeichnet, dass** der Mammalier ein menschliches Wesen ist.

3. Verwendung eines Milchsäurebakterienstammes zur Herstellung eines Medikaments zur Erleichterung der Induktion der Apoptose von Krebszellen, wobei der Stamm *Lactobacillus acidophilus* I-1492 darstellt, der bei der CNCM hinterlegt ist.

4. Verwendung eines Milchsäurebakterienstammes zur Herstellung eines Medikaments zur Behandlung oder Vorbeugung von Krebs, wobei der Stamm *Lactobacillus acidophilus* I-1492 darstellt, der bei der CNCM hinterlegt ist.

5. Die Verwendung nach irgend einem der Ansprüche 1 bis 4, **dadurch gekennzeichnet, dass** der Bakterienstamm in lebender Form vorliegt, oder in nicht-lebender aber intakter Form vorliegt.

6. Die Verwendung nach irgend einem der Ansprüche 1 bis 5, **dadurch gekennzeichnet, dass** außerdem ein Stamm der Spezies *Lactobacillus casei* verwendet wird.

7. Die Verwendung nach irgend einem der Ansprüche 1 bis 6, zusätzlich mit einem Antikrebsmittel.

8. Die Verwendung nach Anspruch 7, **dadurch gekennzeichnet, dass** das Antikrebsmittel 5-Fluoruracil darstellt.

9. Die Verwendung nach irgend einem der Ansprüche 1 bis 8, **dadurch gekennzeichnet, dass** die besagte Krebsart Colonkrebs ist.

10. Zusammensetzung zur Behandlung oder Vorbeugung von Krebs, enthaltend eine wirksame Menge eines Milchsäurebakterienstammes und einen pharmazeutisch akzeptablen Träger, wobei der Stamm *Lactobacillus acidophilus* I-1492 darstellt, der bei der CNCM hinterlegt ist.

11. Die Zusammensetzung nach Anspruch 10, **dadurch gekennzeichnet, dass** der Bakterienstamm in lebender Form vorliegt, oder in nicht-lebender aber intakter Form vorliegt.

12. Die Zusammensetzung nach Anspruch 10 oder 11, **dadurch gekennzeichnet, dass** außerdem ein Stamm der Spezies *Lactobacillus casei* enthalten ist.

13. Die Zusammensetzung nach irgend einem der Ansprüche 10 bis 12, **dadurch gekennzeichnet, dass** sie ein Antikrebsmittel enthält.

14. Die Zusammensetzung nach Anspruch 13, **dadurch gekennzeichnet, dass** das Antikrebsmittel 5-Fluoruracil ist.

15. Die Zusammensetzung nach irgend einem der Ansprüche 10 bis 14, **dadurch gekennzeichnet, dass** die besagte Krebsart Colonkrebs ist.

16. Kit zur Vorbeugung oder Behandlung von Krebs bei Mammaliern, **dadurch gekennzeichnet, dass** es einen Behälter aufweist enthaltend eine Zusammensetzung, die definiert ist nach einem der Ansprüche 10 bis 14.

**FIG. 1**

Stresse oxidatif
Radiations ionisantes
Lésions à l'ADN (p53)

FasL
TNFα

Privation en
facteurs de
croissance

autres

Perforine
Granzyme B

1° Signal

Bcl-2, Bcl-xL
mitochondrie

Mitochondrie
Δψm, cytochrome c
AIF, radicaux libres

2° contrôle

Boucle d'auto
amplification

3° exécution    Caspases

Apoptose

FIG. 2

FIG. 3

## FIG. 4

FIG. 5

## FIG. 6

## FIG. 7

## FIG. 8

Sous-G1

Nombre
d'évènements

250 ug/mL
100 ug/mL
50 ug/mL
25 ug/mL
2.5 ug/mL
Témoin

Fluorescence

FIG. 9

## FIG. 10

## FIG. 11

A) Témoin

B)  5FU (100 µg/mL)

C)  Bactéries vivantes $10^8$

.D)  Bactéries vivantes $10^8$ + 5FU

FIG. 12

| 0h | 6h | 12h | 24h | 36h | 48h |
|---|---|---|---|---|---|

5FU

5FU + bactéries
vivantes

## FIG. 13

FIG. 14

FIG. 15

FIG. 16

5FU

5FU + bactéries
vivantes

5FU + bactéries
chauffées

## FIG. 17

## FIG. 18

FIG. 19

**FIG. 20**

**Expression de Fas Récepteur**

## FIG. 21

**Expression de Fas ligand**

FIG. 22

5FU

5FU + bactéries
vivantes

5FU + bactéries
chauffées

FIG. 23

5FU

5FU + bactéries
vivantes

5FU + bactéries
chauffées

FIG. 24

## FIG. 25

FIG. 26

FIG. 27

FIG. 28.

Ctrl +Serum

Ctrl - Serum (100X)

FIG. 28 (Gm·T)

| 5FU (2.5 μg/ml) | 5FU + S3 | 5FU + S6 | 5FU + S9 |
| 5FU (100 μg/ml) | 5FU + S3 | 5FU + S6 | 5FU + S9 |

EP 1 450 832 B1

Fig. 28 (Con't)

Ctrl
+Serum

Ctrl -
Serum
(630X)

S9

S6

S3

*Fig. 25 (cont'T)*

| | | | |
|---|---|---|---|
| **5FU (2.5 µg/ml)** | **5FU + S3** | **5FU + S6** | **5FU + S9** |
| **5FU (100 µg/ml)** | **5FU + S3** | **5FU + S6** | **5FU + S9** |

FIG. 29

FIG. 30